Europäisches Patentamt

European Patent Office

Office européen des brevets

(11) Publication number: **0 107 053**
**B1**

(12) **EUROPEAN PATENT SPECIFICATION**

(45) Date of publication of patent specification: **13.09.89**

(51) Int. Cl.⁴: **C 07 K 7/00, C 07 K 15/00 //**
**G01N33/53**

(21) Application number: **83109481.8**

(22) Date of filing: **23.09.83**

(54) **Human leukemia virus-related peptides, antibodies of the peptides and a process for production of the antibodies.**

(30) Priority: **30.09.82 JP 171313/82**
**07.01.83 JP 1495/83**
**16.02.83 JP 25233/83**
**23.02.83 JP 30096/83**

(43) Date of publication of application:
**02.05.84 Bulletin 84/18**

(45) Publication of the grant of the patent:
**13.09.89 Bulletin 89/37**

(84) Designated Contracting States:
**CH DE FR GB IT LI NL SE**

(56) References cited:
**FR-A-2 228 496**

**Oroszlan et al., Proc. Natl. Adad. Sci., vol. 79,
pages 1291-1294 "Primary structure analysis of
the major internal protein p-24 of human type
C/T-cell Leukemia virus"**

(73) Proprietor: **JAPANESE FOUNDATION FOR
CANCER RESEARCH
37-1, Kamiikebukuro 1-chome
Toshima-ku Tokyo (JP)**
(73) Proprietor: **OTSUKA PHARMACEUTICAL CO.,
LTD.
9, Kandatsukasacho 2-chome
Chiyoda-ku Tokyo 101 (JP)**

(72) Inventor: **Yoshida, Mitsuaki
No. 3-11-17, Jiyugaoka
Meguro-ku Tokyo (JP)**
Inventor: **Sugano, Haruo
No. 4-8-13, Minami Ogikubo
Suginami-ku Tokyo (JP)**
Inventor: **Shimizu, Fumio
No. 8-71, Kita Okinosu 2-chome
Tokushima-shi Tokushima (JP)**
Inventor: **Tachikawa, Tetsuya
No. 23-4, Aza Nakasu Nakamura Kitajima-cho
Itano-gun Tokushima (JP)**
Inventor: **Ikei, Nobuhiro
No. 463-10, Kagasuno Kawauchi-cho
Tokushima-shi Tokushima (JP)**
Inventor: **Noda, Atunari
No. 463-10, Kagasuno Kawauchi-cho
Tokushima-shi Tokushima (JP)**

Courier Press, Leamington Spa, England.

# EP 0 107 053 B1

(56) References cited:
Kalyanarman et al., Proc. Natl. Acad. Sci., vol. 79, pages 1653-1657 "Natural antibodies to the structural core protein (p-24) of the human T-Cell Leukemia retrovirus"

Seiki et al., Proc. Natl. Acad. Sci. vol. 80, pages 3618-3622 "Human adult T-Cell Leukemia virus: Complete nucleotide sequence of the provirus genome integrated in Leukemia Cell DNA"

Yoshida et al., Proc. Natl. Acad. Sci. vol. 79, pages 2031-2035 "Isolation and characterisation of retrovirus from cell lines of human adult T-Cell Leukemia and its implication in the disease"

Hinuma et al. Int. J. Cancer, vol. 29, pages 631-635 "Antibodies to adult T-Cell Leukemia-virus-associated Antigen (ATLA) in Sera from patients with ATC"

Hoppe et al., Proc. Natl. Acad. Sci. vol. 78, pages 3824-3828 "Prediction of protein antigenic determinants from amino acid sequences"

(72) Inventor: Hashimura, Etsuro
No. 1-3-13, Kitayaso-cho
Tokushima-shi Tokushima (JP)
Inventor: Imagawa, Kenichi
No. 1-83, Aza Kitahari Shinkirai
Kitajima-cho Itano-gun Tokushima (JP)

(74) Representative: Hansen, Bernd, Dr.rer.nat. et al
Hoffmann, Eitle & Partner Patentanwälte
Arabellastrasse 4 Postfach 81 04 20
D-8000 München 81 (DE)

# EP 0 107 053 B1

**Description**

The present invention relates to novel peptide associated with human leukemia virus (hereafter also referred to as ATLV short for adult T-cell leukemia virus or as HTLV short for human T-cell leukemia virus) and more particularly, to peptides associated with such viral infections as well as mature T-cell leukemia or lymphoma such as adult T-cell leukemia, cutaneous T-cell lymphoma, etc.

In the specification, amino acids, peptides, protective groups, active groups, nucleotides and others are expressed pursuant to the IUPAC Rules, the IUB Rules or common symbols established in the art when they are abbreviated; examples of which are given below. In case that optical isomers can be present with respect to amino acids or the like, an L-form is meant unless otherwise indicated.

| | |
|---|---|
| Ser: | serine |
| Leu: | leucine |
| Thr: | threonine |
| Asn: | asparagine |
| Gln: | glutamine |
| Glu: | glutamic acid |
| Lys: | lysine |
| Pro: | proline |
| Val: | valine |
| Trp: | tryptophane |
| His: | histidine |
| Asp: | aspartic acid |
| Gly: | glycine |
| Ile: | isoleucine |
| Ala: | alanine |
| Tyr: | tyrosine |
| Met: | methionine |
| Phe: | phenylalanine |
| Arg: | arginine |
| Cys: | cysteine |
| A: | adenine |
| T: | thymine |
| G: | guanine |
| C: | cytosine |
| Tos: | p-toluenesulfonyl group |
| Boc: | tert-butoxycarbonyl group |
| ONP: | p-nitrophenoxy group |
| Bzl: | benzyl group |
| OBzl: | benzyloxy group |
| Cl$_2$-Bzl: | 2,6-dichlorobenzyl group |
| Cl-Z: | 2-chlorobenzyloxycarbonyl group |

Human leukemia virus has been isolated from a patient with adult T-cell leukemia (ATL) and has been shown to be closely associated with the disease. The provirus gene integrated in host cell DNA was molecularly cloned and the complete nucleotide sequence was determined by M. Yoshida and H. Sugano, the present inventors.

The present invention has been accomplished based on the aforesaid basic information and is directed to such virus-related peptides aiming at diagnosis of such virus infections as well as a process for preparation of and a method of measurement for a specific antibody to these peptides. The nucleotide sequence coding for a precursor of core (gag) proteins of the thus determined virus gene described above is shown in Table 1 below.

3

EP 0 107 053 B1

TABLE 1

| ATG | GGC | CAA | ATC | TTT | TCC | CGT | AGC | GCT |
|-----|-----|-----|-----|-----|-----|-----|-----|-----|
| Met | Gly | GLN | Ile | Phe | Ser | Arg | Ser | Ala |

| AGC | CCT | ATT | CCG | CGA | CCG | CCC | CGG | GGG |
|-----|-----|-----|-----|-----|-----|-----|-----|-----|
| Ser | Pro | Ile | Pro | Arg | Pro | Pro | Arg | Gly |

| CTG | GCC | GCT | CAT | CAC | TGG | CTT | AAC | TTC |
|-----|-----|-----|-----|-----|-----|-----|-----|-----|
| Leu | Ala | Ala | His | His | Trp | Leu | Asn | Phe |

| CTC | CAG | GCG | GCA | TAT | CGC | CTA | GAA | CCC |
|-----|-----|-----|-----|-----|-----|-----|-----|-----|
| Leu | Gln | Ala | Ala | Tyr | Arg | Leu | Glu | Pro |

| GGT | CCC | TCC | AGT | TAC | GAT | TTC | CAC | CAG |
|-----|-----|-----|-----|-----|-----|-----|-----|-----|
| Gly | Pro | Ser | Ser | Tyr | Asp | Phe | His | Gln |

| TTA | AAA | AAA | TTT | CTT | AAA | ATA | GCT | TTA |
|-----|-----|-----|-----|-----|-----|-----|-----|-----|
| Leu | Lys | Lys | Phe | Leu | Lys | Ile | Ala | Leu |

| GAA | ACA | CCG | GCT | CGG | ATC | TGT | CCC | ATT |
|-----|-----|-----|-----|-----|-----|-----|-----|-----|
| Glu | Thr | Pro | Ala | Arg | Ile | Cys | Pro | Ile |

| AAC | TAC | TCC | CTC | CTA | GCC | AGC | CTA | CTC |
|-----|-----|-----|-----|-----|-----|-----|-----|-----|
| Asn | Tyr | Ser | Leu | Leu | Ala | Ser | Leu | Leu |

| CCA | AAA | GGA | TAC | CCC | GGC | CGG | GTG | AAT |
|-----|-----|-----|-----|-----|-----|-----|-----|-----|
| Pro | Lys | Gly | Tyr | Pro | Gly | Arg | Val | Asn |

| GAA | ATT | TTA | CAC | ATA | CTC | ATC | CAA | ACC |
|-----|-----|-----|-----|-----|-----|-----|-----|-----|
| Glu | Ile | Leu | His | Ile | Leu | Ile | Gln | Thr |

| CAA | GCC | CAG | ATC | CCG | TCC | CGT | CCC | GCG |
|-----|-----|-----|-----|-----|-----|-----|-----|-----|
| Gln | Ala | Gln | Ile | Pro | Ser | Arg | Pro | Ala |

| CCA | CCG | CCG | CCG | TCA | TCC | CCC | ACC | CAC |
|-----|-----|-----|-----|-----|-----|-----|-----|-----|
| Pro | Pro | Pro | Pro | Ser | Ser | Pro | Thr | His |

| GAC | CCC | CCG | GAT | TCT | GAT | CCA | CAA | ATC |
|-----|-----|-----|-----|-----|-----|-----|-----|-----|
| Asp | Pro | Pro | Asp | Ser | Asp | Pro | Gln | Ile |

| CCC | CCT | CCC | TAT | GTT | GAG | CCT | ACG | GCC |
|-----|-----|-----|-----|-----|-----|-----|-----|-----|
| Pro | Pro | Pro | Tyr | Val | Glu | Pro | Thr | Ala |

| CCC | CAA | GTC | CTT | CCA | GTC | ATG | CAT | CCA |
|-----|-----|-----|-----|-----|-----|-----|-----|-----|
| Pro | Gln | Val | Leu | Pro | Val | Met | His | Pro |

| CAT | GGT | GCT | CCT | CCT | AAC | CAT | CGC | CCA |
|-----|-----|-----|-----|-----|-----|-----|-----|-----|
| His | Gly | Ala | Pro | Pro | Asn | His | Arg | Pro |

| TGG | CAA | ATG | AAA | GAC | CTA | CAG | GCC | ATT |
|-----|-----|-----|-----|-----|-----|-----|-----|-----|
| Trp | Gln | Met | Lys | Asp | Leu | Gln | Ala | Ile |

| AAG | CAA | GAA | GTC | TCC | CAA | GCA | GCC | CCT |
|-----|-----|-----|-----|-----|-----|-----|-----|-----|
| Lys | Gln | Glu | Val | Ser | Gln | Ala | Ala | Pro |

| GGG | AGC | CCC | CAG | TTT | ATG | CAG | ACC | ATC |
|-----|-----|-----|-----|-----|-----|-----|-----|-----|
| Gly | Ser | Pro | Gln | Phe | Met | Gln | Thr | Ile |

| CGG | CTT | GCG | GTG | CAG | CAG | TTT | GAC | CCC |
|-----|-----|-----|-----|-----|-----|-----|-----|-----|
| Arg | Leu | Ala | Val | Gln | Gln | Phe | Asp | Pro |

| ACT | GCC | AAA | GAC | CTC | CAA | GAC | CTC | CTG |
|-----|-----|-----|-----|-----|-----|-----|-----|-----|
| Thr | Ala | Lys | Asp | Leu | Gln | Asp | Leu | Leu |

4

## TABLE 1 (contd.)

| | | | | | | | | |
|---|---|---|---|---|---|---|---|---|
| CAG Gln | TAC Tyr | CTT Leu | TGC Cys | TCC Ser | TCC Ser | CTC Leu | GTG Val | GCT Ala |
| TCC Ser | CTC Leu | CAT His | CAC His | CAG Gln | CAG Gln | CTA Leu | GAT Asp | AGC Ser |
| CTT Leu | ATA Ile | TCA Ser | GAG Glu | GCC Ala | GAA Glu | ACC Thr | CGA Arg | GGT Gly |
| ATT Ile | ACA Thr | GGT Gly | TAT Tyr | AAC Asn | CCA Pro | TTA Leu | GCC Ala | GGT Gly |
| CCC Pro | CTC Leu | CGT Arg | GTC Val | CAA Gln | GCC Ala | AAC Asn | AAT Asn | CCA Pro |
| CAA Gln | CAA Gln | CAA Gln | GGA Gly | TTA Leu | AGG Arg | CGA Arg | GAA Glu | TAC Tyr |
| CAG Gln | CAA Gln | CTC Leu | TGG Trp | CTC Leu | GCC Ala | GCC Ala | TTC Phe | GCC Ala |
| GCC Ala | CTG Leu | CCG Pro | GGG Gly | AGT Ser | GCC Ala | AAA Lys | GAC Asp | CCT Pro |
| TCC Ser | TGG Trp | GCC Ala | TCT Ser | ATC Ile | CTC Leu | CAA Gln | GGC Gly | CTG Leu |
| GAG Glu | GAG Glu | CCT Pro | TAC Tyr | CAC His | GCC Ala | TTC Phe | GTA Val | GAA Glu |
| CGC Arg | CTC Leu | AAC Asn | ATA Ile | GCT Ala | CTT Leu | GAC Asp | AAT Asn | GGG Gly |
| CTG Leu | CCA Pro | GAA Glu | GGC Gly | ACG Thr | CCC Pro | AAA Lys | GAC Asp | CCC Pro |
| ATC Ile | TTA Leu | CGT Arg | TCC Ser | TTA Leu | GCC Ala | TAC Tyr | TCC Ser | AAT Asn |
| GCA Ala | AAC Asn | AAA Lys | GAA Glu | TGC Cys | CAA Gln | AAA Lys | TTA Leu | CTA Leu |
| CAG Gln | GCC Ala | CGA Arg | GGA Gly | CAC His | ACT Thr | AAT Asn | AGC Ser | CCT Pro |
| CTA Leu | GGA Gly | GAT Asp | ATG Met | TTG Leu | CGG Arg | GCT Ala | TGT Cys | CAG Gln |
| ACC Thr | TGG Trp | ACC Thr | CCC Pro | AAA Lys | GAC Asp | AAA Lys | ACC Thr | AAA Lys |
| GTG Val | TTA Leu | GTT Val | GTC Val | CAG Gln | CCT Pro | AAA Lys | AAA Lys | CCC Pro |
| CCC Pro | CCA Pro | AAT Asn | CAG Gln | CCG Pro | TGC Cys | TTC Phe | CGG Arg | TGC Cys |
| GGG Gly | AAA Lys | GCA Ala | GGC Gly | CAC His | TGG Trp | AGT Ser | CGG Arg | GAC Asp |
| TGC Cys | ACT Thr | CAG Gln | CCT Pro | CGT Arg | CCC Pro | CCC Pro | CCC Pro | GGG Gly |

TABLE 1 (contd.)

| CCA | TGC | CCC | CTA | TGT | CAA | GAC | CCA | ACT |
|-----|-----|-----|-----|-----|-----|-----|-----|-----|
| Pro | Cys | Pro | Leu | Cys | Gln | Asp | Pro | Thr |
| CAC | TGG | AAG | CGA | GAC | TGC | CCC | CGC | CTA |
| His | Trp | Lys | Arg | Asp | Cys | Pro | Arg | Leu |
| AAG | CCC | ACT | ATC | CCA | GAA | CCA | GAG | CCA |
| Lys | Pro | Thr | Ile | Pro | Glu | Pro | Glu | Pro |
| GAG | GAA | GAT | GCC | CTC | CTA | TTA | GAC | CTC |
| Glu | Glu | Asp | Ala | Leu | Leu | Leu | Asp | Leu |
| CCC | GCT | GAC | ATC | CCA | CAC | CCA | AAA | AAC |
| Pro | Ala | Asp | Ile | Pro | His | Pro | Lys | Asn |
| TCC | ATA | GGG | GGG | GAG | GTT | | | |
| Ser | Ile | Gly | Gly | Glu | Val | | | |

From Table 1 above, it is shown that the precursor of core proteins is composed of 429 amino acids. In light of the structure of the terminus p-24 previously reported (*Proc. Natl. Acad. Sci. U.S.A.,* vol. 79, pp. 1291—1294 (1982)), it was expected that the precursor would be further cleaved to form core proteins having the termini p-14, p-24 and p-10.

Based on the foregoing viewpoint, the present inventors have found specific peptides which can be haptens of proteins (core proteins) associated with human leukemia virus described above and have accomplished the present invention.

The present invention relates to a human leukemia virus-related peptide selected a peptide represented by the general formula (1):

$$R-Pro-Val-Met-His-Pro-His-Gly-Ala-Pro-OH \quad (1)$$

wherein R is a hydrogen atom or a group of the formula H-Tyr-, in which the Tyr moiety may be labelled with radioactive iodine;

a peptide represented by the formula (2):

$$H-Tyr-Val-Glu-Pro-Thr-Ala-Pro-Gln-Val-Leu-OH \quad (2)$$

a peptide represented by the general formula (3):

$$R-Ile-Pro-His-Pro-Lys-Asn-Ser-Ile-Gly-Gly-Glu-Val-OH \quad (3)$$

wherein R is the same as defined above;

a peptide represented by the general formula (4):

$$R-Thr-Trp-Thr-Pro-Lys-Asp-Lys-Thr-Lys-Val-Leu-OH \quad (4)$$

wherein R is the same as defined above;

a peptide represented by the formula (5):

$$H-Val-Val-Gln-Pro-Lys-Lys-Pro-Pro-Pro-Tyr-OH \quad (5)$$

in which the Tyr moiety may be labelled with radioactive iodine;

a peptide represented by the general formula (6):

$$R-Met-Gly-Gln-Ile-Phe-Ser-Arg-Ser-Ala-Ser-Pro-OH \quad (6)$$

wherein R is the same as defined above; and

a peptide represented by the formula (7):

$$H-Tyr-Pro-Glu-Gly-Thr-Pro-Lys-Asp-Pro-Ile-Leu-Arg-Ser-Leu-Arg-Ser-Leu-OH \quad (7)$$

in which the Tyr moiety may be labelled with radioactive iodine.

Furthermore, the invention comprises antisera containing an antibody of a human leukemia virus-related peptide obtainable by centrifuging the blood collected from a mammal, to which an antigen

6

prepared by reacting a human leukemia virus-related peptide selected from the peptides (1) to (7) as defined above as a hapten, with a carrier in the presence of a hapten-carrier binding agent, has been administered.

In the above-mentioned antibody-containing sera said carrier is preferably selected from an animal serum albumin, an animal serum globulin, an animal thyroglobulin, an animal hemoglobulin, an animal hemocyanin, an ascaris extract, a polylysine, a polyglutamic acid, a lysine-glutamic acid copolymer, and a copolymer containing lysine or ornithine; and said hapten-carrier binding agent is selected from a diazonium compound, an aliphatic dialdehyde, a dimaleimide compound, a maleimidocarboxyl - N - hydroxysuccinimide ester and carbodiimide.

Preferably said mammal is rabbit or guinea pig.

The invention also comprises a method for preparing an antibody of human leukemia virus-related peptide-containing sera comprising the following steps:

reacting a human leukemia virus-related peptide selected from the peptides (1) to (7) as defined above as a hapten, with a carrier in the presence of a hapten-carrier binding agent to form an antigen,

administering the resulting antigen to a mammal to form an antibody,

centrifuging the blood collected from the animals, and

isolating the antibody-containing serum from the blood.

Fig. 1 is a curve showing the affinity of Peptide A to the antibody of the present invention.

Fig. 2 is a curve showing the reactivity (specificity) of the antibody of the present invention to ATL-associated antigen (ATLA).

Fig. 3 is a curve showing the reactivity (specificity) of the antibody of the present invention to ATL-associated antigen (ATLA) and to Peptide C.

Fig. 4 is a curve showing the reactivity (specificity) of the antibody of the present invention to ATL-associated antigen (ATLA) and to Peptide F.

Fig. 5 is a curve showing the reactivity (specificity) of the antibody of the present invention to ATL-associated antigen (ATLA) and to Peptide H.

The peptides of the present invention shown by formulae (1) to (7) described above can all be easily prepared by simple operations utilizing easily accessible, commercially available amino acids. From each of the peptides, antigens can be prepared using them as haptens. From the thus obtained antigens, antibodies having a specific reactivity with virus-associated proteins can be obtained. Particularly when the peptides shown by formula (1) are employed, antibodies having a reactivity particularly with p-24 can be obtained. These specific antibodies are usable for purification of virus-associated proteins, by binding these antibodies to carriers for use of, e.g., affinity chromatography, and utilizing the bound antibodies in the chromatography. The specific antibodies can also be utilized as specific antibodies in various immunological measurements of such virus-associated proteins. Thus, these antibodies are useful for diagnosis of human leukemia virus infections and further for diagnosis, studies, etc. of mature T-cell leukemia or lymphoma such as adult T-cell leukemia, cutaneous T-cell lymphoma, as well as diseases related thereto.

The peptides of the present invention represented by the general formulae (1) through (7) can be prepared by conventional processes for synthesizing peptides; more specifically, using processes as described in Schroder and Luhke, *The Peptides*, vol. 1 (1966), published by Academic Press, New York, U.S.A., or Izumiya et al., *Synthesis of Peptides*, (1975), published by Maruzen Publishing Co., Ltd., for example, an azide process, a chloride process, an acid anhydride process, a mixed anhydride process, a DCC process, an active ester process (a p-nitrophenyl ester process, an N - hydroxysuccimimide ester process, a cyanomethyl ester process), a process using a Woodward reagent K, a carbodiimidazole process, an oxidative reduction process and a DCC/additive (HONB, HOBt, HOSu) process. Solid phase and liquid phase syntheses are both applicable to the foregoing processes.

The peptides of the present invention are prepared in accordance with the aforesaid processes for synthesizing ordinary polypeptides, generally either by a so called stepwise process which comprises condensing an amino acid to the terminal amino acid one by one in sequence, or by coupling fragments divided into several groups to the terminal amino acid. In more detail, for example, in case that a solid phase synthesis is adopted, the C terminal amino acid is bound to an insoluble carrier through its carboxyl group. The insoluble carrier is not particularly limited as long as it has a binding capability to a reactive carboxyl group. Examples of such insoluble carriers include halogenomethyl resins such as chloromethyl resin and bromomethyl resin; hydroxymethyl resins, phenol resins and tert - alkyloxycarbonyhydrazided resins.

After the amino protective group is removed, an amino group-protected amino acid is bound in sequence in accordance with the amino acid sequence shown by general formulae (1) through (7) through condensation of its reactive amino group and the reactive carboxyl group, in sequence, to synthesize step by step. After synthesizing the complete sequence, the peptide is split off from the insoluble carrier to produce the protein.

In the foregoing process, it is preferred that respective amino acids of histidine, arginine, tyrosine, glutamic acid, threonine, lysine, aspartic acid and serine be protected at the side chain functional groups. These functional groups at the side chain are protected with ordinary protective groups which are split off after completion of the reaction. The functional groups which take part in the reaction are generally activated. These processes are known and reagents used in these processes are also appropriately chosen from known ones.

Examples of protective groups for amino groups include a benzyloxycarbonyl, Boc, tert - amyloxy-carbonyl, isobornyloxycarbonyl, p - methoxybenzyloxycarbonyl, Cl-Z, adamantyloxycarbonyl, trifluoro-acetyl, phthalyl, formyl, o - nitrophenylsulfenyl and diphenylphosphinothiol group.

Examples of protective groups for the imino group of histidine include a Tos, Bzl, benzyloxycarbonyl and a trityl group.

Examples of protective groups for arginine include a Tos, nitro and a benzyloxycarbonyl group.

Examples of protective groups for the hydroxy groups of serine and threonine include a Bzl, tert-butyl, acetyl and a tetrahydropyranyl group.

Examples of protective groups for the hydroxy group of tyrosine include a Bzl, $Cl_2$-Bzl, benzyloxy-carbonyl, acetyl and a·Tos group.

Examples of protective groups for the amino group of lysine include a benzyloxycarbonyl, Cl-Z, $Cl_2$-Bzl, Boc and a Tos group.

Protection for the carboxyl groups of glutamic acid and aspartic acid includes esterification of the carboxylic acids with benzyl alcohol, methanol, ethanol and tert-butanol.

Examples of activated carboxyl groups include the corresponding acid chlorides, acid anhydrides or mixed acid anhydrides, azides, active esters (esters with pentachlorophenol, p - nitrophenol, N - hydroxysuccinimide, N - hydroxybenzotriazole or N - hydroxy - 5 - norbornene - 2,3 - dicarboxydi-imide).

In some cases, the peptide bond forming reaction may also be carried out in the presence of carbodiimide reagents such as dicyclohexylcarbodiimide, carbodiimidazole or tetraethylpyrophosphine.

Hereafter, the preparation of the peptides in accordance with the present invention will be explained more specifically with reference to reaction equations below, as an example.

(Reaction Equations 1)

$$A\text{-Tyr-OH} \qquad\qquad\qquad (a)$$
$$\downarrow$$
$$A\text{-Tyr-R}^1 \qquad\qquad\qquad (b)$$
$$\downarrow$$
$$H\text{-Tyr-R}^1 \qquad\qquad\qquad (c)$$
$$\bigg| \quad A\text{-Pro-OH} \qquad\qquad (d)$$
$$\downarrow$$
$$A\text{-Pro-Tyr-R}^1 \qquad\qquad (e)$$
$$\downarrow \ \downarrow \ \downarrow$$
$$A\text{-Val-Val-Gln-Pro-Lys-Lys-Pro-Pro-Pro-Tyr-R}^1 \qquad (f)$$
$$\downarrow$$
$$H\text{-Val-Val-Gln-Pro-Lys-Lys-Pro-Pro-Pro-Tyr-OH} \qquad (5)$$

wherein A represents a protective for an amino group and $R^1$ represents an insoluble carrier.

Of the foregoing, preferred A includes Boc, a benzyloxycarbonyl group or a p - methoxybenzyloxy-carbonyl group and preferred $R^1$ includes chloromethylated polystyrene or the like, respectively.

In case that amino acids used possess functional groups at the side chain thereof which do not participate in each of the reactions, the amino acids are protected by the protective groups described above in a conventional manner and the protective groups are split off at the same time as splitting-off of the insoluble carrier $R^1$.

In the processes described above, the reaction of the amino acid (a) with the insoluble carrier $R^1$ (b) is carried out by utilizing the reactive carboxyl group of the amino acid (a) and binding it to $R^1$ in a conventional manner. The reaction is effected in an appropriate solvent in the presence of basic compounds, e.g., triethylamine, potassium tert-butoxide, cesium carbonate and cesium hydroxide in the case of using, e.g., chloromethylated polystyrene. Examples of solvents include dimethylformamide (DMF), dimethylsulfoxide (DMSO), pyridine, chloroform, dioxan, dichloromethane, tetrahydrofuran, N - methylpyrrolidone and hexamethylphosphoric acid triamide or a mixture solvent thereof. The above reaction is generally completed at temperatures of about 0 to about 85°C, preferably at 25 to 80°C for several minutes to about 24 hours. It is preferred that an amount of the amino acid to the insoluble carrier be set forth such that the former is employed in an excess amount, generally 1 to 3 time equivalents per 1 equivalent of the latter.

Splitting of the protective group A for the thus obtained amino acid shown by general formula (b) is carried out in a conventional manner. For example, there are hydrogenation using catalysts such as palladium and palladium black, a reduction method involving reduction with metallic sodium in liquid ammonia; acidolysis using strong acids such as trifluoroacetic acid, hydrogen chloride, hydrogen fluoride, methanesulfonic acid and hydrogen bromide. The hydrogenation using the foregoing catalysts can be carried out, e.g., under hydrogen pressure of 1 atm at temperatures of 0 to 40°C. It is preferred that the catalyst be used generally in an amount of about 100 mg to about 1 g. The reaction is generally completed within about 1 to about 48 hours. The acidolysis described above is carried out generally at temperatures of about 0 to about 30°C, preferably 0 to 20°C for about 15 minutes to about 1 hour, in the absence of any solvent. It is preferred that the acid be used in an amount of generally 5 to 10 times that of the raw compound. In case that the protective group A alone is wished to be split off in the acidolysis, it is preferred to use trifluoroacetic acid or hydrogen chloride as the acid. The aforesaid reduction with metallic sodium in liquid ammonia can be carried out generally at temperatures of about −40 to about −70°C, using metallic sodium in such an amount that is colored to permanent blue for about 30 seconds to about 10 minutes.

The reaction of the subsequently obtained amino acid in a solid phase shown by general formula (c) and the amino acid (d) (or a derivative thereof in which the carboxyl group is activated) is carried out in the presence of a solvent. As solvents, there can be used various known solvents conventionally used in peptide condensation, for example, anhydrous, dimethylformamide, dimethylsulfoxide, pyridine, chloroform, dioxan, dichloromethane, tetrahydrofuran, ethyl acetate, N - methylpyrrolidone, hexamethylphosphoric acid triamide or a solvent mixture thereof. The reaction can also be conducted, if necessary and desired, in the presence of reagents conventionally employed in ordinary peptide bond forming reactions, for example, dehydrating and condensing agents such as carbodiimides, e.g., N,N - dicyclohexylcarbodiimide (DCC), N - ethyl - N' - dimethylaminocarbodiimide, 1 - ethyl - 3 - diisopropylaminocarbodiimide and 1 - cyclohexyl - 3 - (2 - morpholinyl - 4 - ethyl)carbodiimide. While there is no particular limitation to the proportion of the amino acid (c) to the amino acid (d) to be used, it is preferred that the latter be employed in an amount of an equimolar amount to 10 time moles that of the former, preferably from an equimolar amount to 5 time moles. There is no particular limitation to the amount of the dehydrating and condensing agent to be used, either; the agent is generally employed preferably in an equimolar amount to that of the amino acid (d). The reaction temperature is suitably chosen from a normal range conventionally used for peptide bond forming reactions, generally from the range of about −40 to about 60°C, preferably from the range of about −20 to about 40°C. The reaction time is generally set forth for about several minutes to about 30 hours.

The thus obtained peptide shown by general formula (e) is, after splitting-off the protective group A as described above, condensed in sequence with each of the amino acids, A-Pro-OH, A-Pro-OH, A-Lys-OH, A-Lys-OH, A-Pro-OH, A-Gln-OH, A-Val-OH and A-Val-OH, in accordance with the amino acid sequence shown by general formula (5) or, derivatives thereof wherein the functional groups at the side chain are protected or the carboxyl groups are activated. Thus, the peptide shown by general formula (e) can be introduced into the peptide represented by general formula (f). These condensation and splitting-off of the protective group A are carried out in a manner similar to those described above.

The thus obtained peptide (f) can be introduced into the peptide shown by general formula (5) by splitting of the protective group A, splitting-off of the protective groups of the amino acid at the side chain thereof and removing the insoluble carrier R$^1$. Here the removal of the protective groups at the side chain functional groups and the insoluble carrier R$^1$ can be carried out in a manner similar to the splitting off of the protective group A; in this case, it is preferred to use hydrogen fluoride or hydrogen bromide as the acid. All of the amino acids used in the aforesaid processes may be those commercially available.

The thus produced peptide of the present invention shown by formula (5) can be isolated and purified from the reaction mixture by means of peptide separation, e.g., extraction, distribution and column chromatography.

Further, the peptides represented by general formulae (1) to (4), (6) and (7) can also be prepared in a manner similar to the process described above.

The thus obtained peptides of the present invention are utilizable as labelled antigens employed in radioimmunology (RIA) or enzymeimmunoassay (EIA), by introducing thereto radioactive substances such as $^{125}$I or $^{131}$I; various enzyme reagents such as peroxidase (POX), chymotripsinogen, procarboxypeptidase, glycerolaldehyde - 3 - phosphate dehydrogenase, amylase, phospholyrase, D-Nase, P-Nase, β - galactosidase, glucose - 6 - phosphate dehydrogenase and ornithine decarboxylase. The introduction of the above radioactive substance can be effected in a conventional manner. For example, the introduction of radioactive iodine can be carried out by the oxidative iodination method (W. M. Hunter and F. C. Greenwood, Nature, 194, 495 (1962), Biochem. J., 89, 144 (1963)) using chloramine T. The introduction of enzyme reagents can be conducted by known methods such as conventional couplings, e.g., the B. F. Erlanger, et al method (*Acta Endocrinol. Suppl., 168*, 206 (1972)) or the M. H. Karol et al method (Proc. Natl. Acd. Sci. U.S.A., 57, 713 (1967).

Hereafter processes for production of antigens using the peptides of the present invention as haptens will be described in detail.

The aforesaid antigens are prepared by using the peptides of the present invention as haptens and reacting the peptides with a suitable carrier in the presence of a hapten-carrier binding agent. In this case, natural and synthetic proteins having a high molecular weight which are conventionally employed in the preparation of antigens can be widely employed as carriers to be bound to haptens. Examples of such carriers include albumins of animal sera such as horse serum albumin, bovine serum albumin, rabbit serum albumin, human serum albumin and sheep serum albumin; globulins of animal sera such as horse serum globulin, bovine serum globulin, rabbit serum globulin, human serum globulin and sheep serum globulin; thyroglobulins of animals such as horse thyroglobulin, bovine thyroglobulin, rabbit thyroglobulin, human thyroglobulin and sheep thyroglobulin; hemoglobulins of animals such as horse hemoglobulin, bovine hemoglobulin, rabbit hemoglobulin, human hemoglobulin and sheep hemoglobulin; hemocyanins of animals such as Keyhole limpet hemocyanin (KLH); proteins extracted from ascaris (ascaris extracts, those described in Japanese Patent Application (OPI) No. 16414/81, *J. Immun., 111*, 260—268 (1973), ibid., *122*, 302—308 (1979), ibid., *98*, 893—900 (1967) and *Am. J. Physiol., 199*, 575—578 (1960), or purified products thereof); polylysine, polyglutamic acid, lysine-glutamic acid copolymers, copolymers containing lysine or ornithine.

As hapten-carrier binding agents, those conventionally employed in the preparation of antigens can be widely employed. Specific examples of these agents include diazonium compounds for cross linking tyrosine, histidine, or tryptophane, e.g., bisdiazotized benzidine (BDB) and bisdiazotized - 3,3' - dianisidine (BDD); aliphatic dialdehydes for cross linking an amino group with an amino group, e.g., glyoxal, malonedialdehyde, glutaraldehyde, succinaldeyde or adipoaldehyde; dimaleimide compounds for cross linking a thiol group with a thiol group, e.g., N,N' - o - phenylenedimaleimide and N,N' - m - phenylenedimaleimide; maleimidocarboxyl - N - hydroxysuccinimide esters for cross linking an amino group with a thiol group, e.g., metamaleimidobenzoyl - N - hydroxysuccinimide ester and 4 - (maleimidomethyl) - cyclohexane - 1 - carboxyl - N' - hydroxysuccinimide ester; agents used in conventional peptide bond forming reactions for amide-binding an amino group with a carboxyl group, e.g., dehydrated and condensing agents such as carbodiimides, e.g., N,N - dicyclohexylcarbodiimide, N - ethyl - N' - dimethylaminocarbodiimide, 1 - ethyl - 3 - diisopropylaminocarbodiimide and 1 - cyclohexyl - 3 - (2 - morpholinyl - 4 - ethyl)carbodiimide. As the foregoing hapten-carrier binding agent, it is also possible to use diazonium aryl carboxylic acids such as p - diazonium phenylacetic acid with conventional peptide bond forming agents such as the dehydrating and condensing agents described above in combination.

The reaction for preparing the antigens described above is carried out in an aqueous solution or a conventional buffer solution having pH of 7 to 10, preferably in a buffer solution having pH of 8 to 9, at temperatures of about 0 to 40°C, preferably around room temperature. The reaction is generally completed within about 1 to about 24 hours, preferably 3 to 5 hours. Representative examples of buffer solutions which can be used in the above process include:

0.2M sodium hydroxide—0.2M boric acid—0.2M potassium chloride buffer solution

0.2M sodium carbonate—0.2M boric acid—0.2M potassium chloride

0.05M sodium tetraborate—0.2M boric acid—0.05M sodium chloride buffer solution

0.1M dihydrogen potassium phosphate—0.05M sodium tetraborate buffer solution

In the above, proportions of the hapten, hapten-carrier binding agent and carrier can be appropriately determined but it is preferred that the carrier be employed in an amount of about 1 to about 6 times, preferably about 1 to about 5 times and the hapten-carrier binding agent be employed in an amount of about 5 to about 10 times, the weight of the hapten. By the above reaction, the carrier is bound to the hapten via the hapten-carrier binding agent to obtain a desired antigen composed of a peptide-carrier complex.

After completion of the reaction, the thus obtained antigen can easily be isolated and purified by means of a dialysis method, a gel filtration method or a fractionation precipitation method.

The preparation of an antibody using the antigen is carried out by administering the aforesaid antigen to mammals to thereby produce a desired antibody in vivo and collecting the antibody.

While there is no particular limitation to mammals provided for the preparation of antibodies, it is generally preferred to use rabbits or guinea pigs. In the production of antibodies, a definite amount of the antigen obtained as described above is diluted with a physiological saline solution to a suitable concentration and the resulting dilution is mixed with a complete Freund's adjuvant to prepare a suspension. The suspension is administered to mammals. For example, the aforesaid suspension is intracutaneously administered (1 to 5 mg/time as the amount of the antigen) to rabbit. Then the suspension is administered every two weeks over a period of 2 to 10 months, preferably 4 to 6 months to effect immunization. The collection of the antibody is carried out by collecting blood from the immunized animal after the passage of 1 to 2 weeks subsequent to the final administration, centrifuging the blood and isolating serum from the blood. According ot this procedure, an antibody having an excellent specificity to the antigen used can be collected and used for assaying human leukemia virus-related proteins utilizing RIA and EIA.

For purposes of explaining the present invention in more detail, preparations of the peptides shown by general formulae (1) to (7), antigens obtained from the peptides and antibodies will be shown by way of examples but the present invention is not deemed to be limited thereto.

Rf values in the respective preparation examples were measured using solvent mixtures described below by means of thin layer chromatography on silica gel.

$Rf^1$ . . . n-butanol—acetic acid—water (4:1:5)

$Rf^2$ . . . n-butanol—acetic acid—pyridine—water (15:3:10:12)

(Preparation of peptides)

Synthesis Example 1

(1) In 14 ml of a DMSO solution of 5.88 milliequivalents of potassium tert-butoxide 1.42 g of a Boc-Pro-OH was dissolved and 5 g of chloromethylated polystyrene resin (Protein Research Promotion Foundation) was added to the solution. The mixture was reacted at 80°C for 30 minutes. After thoroughly washing the resin subsequently with DMSO, 50% acetic acid/chloroform and methylene chloride, the resin was dried under reduced pressure to obtain 5.27 g of Boc-Pro-resin.

A part of the Boc-Pro-resin was hydrolyzed and subjected to amino acid analysis. The results indicate that the product contained 0.36 mmol of the amino acid/g of the resin.

(2) After washing 4 g of the Boc-Pro-resin obtained in (1) above three times with 30 ml of chloroform, the resin was added to 30 ml of a chloroform solution of 50% trifluoroacetic acid (TFA) and the mixture was reacted at room temperature for 20 minutes. The reaction mixture was washed once with 30 ml of chloroform, 5 times with 30 ml of methylene chloride, 3 times with 30 ml of a methylene chloride solution of 10% triethyl amine and then 6 times with 30 ml of methylene chloride to obtain H-Pro-resin.

To 25 ml of a solution of 0.68 g of Boc-Ala-OH in methylene chloride the H-Pro-resin described above was added and 5 ml of a solution of 0.74 g of DCC in methylene chloride was then added to the resulting mixture. The mixture was reacted at room temperature for 2 hours. After washing the resin 6 times with 30 ml of methylene chloride, the resin was added to 25 ml of a methylene chloride solution of 0.68 g of Boc-Ala-OH and 0.55 g of 1 - hydroxybenzotriazole. Then, 5 ml of a methylene chloride solution of 0.74 g of DCC was added thereto and the resulting mixture was again reacted in a similar manner (double coupling). The resin was thoroughly washed with methylene chloride to obtain Boc-Ala-Pro-resin.

(3) In a manner similar to (2) described above, des - tert - butoxycarbonylation (hereafter simply referred to as des-Boc) of the Boc-Ala-Pro-resin was conducted and amino acids described below were then condensed in order, each followed by conducting des-Boc.

| | |
|---|---|
| Boc-Gly-OH | 0.63 g |
| Boc-His-OH<br>\|<br>Tos | 1.47 g |
| Boc-Pro-OH | 0.77 g |
| Boc-His-OH<br>\|<br>Tos | 1.47 g |
| Boc-Met-OH | 0.90 g |
| Boc-Val-OH | 0.78 g |
| Boc-Pro-OH | 0.77 g |

Thus 2.2 g of H - Pro - Val - Met - His(Tos) - Pro - His(Tos) - Gly - Ala - Pro - resin. To the thus obtained resin 2 ml of anisole, 25 ml of hydrogen fluoride and 0.5 ml of ethanedithiol were added. After reacting the mixture at −20°C for 30 minutes and then at 0°C for 30 minutes, a peptide was obtained. Purification of the peptide using Sephadex G-25 (manufactured by Pharmacia Co., Ltd.; eluting liquid, 10% aqueous acetic acid solution), CM-Sephadex C-25 (manufactured by Pharmacia Co., Ltd.; eluting liquid, 0.1—0.5M aqueous ammonium acetate solution; concentration gradient, pH=4) and then HPLC (eluting liquid, 0.01% aqueous ammonium acetate solution:acetonitrile=70:30) using μ-Pondapack C-18 (manufactured by Waters Co., Ltd.) gave 341 mg of H - Pro - Val - Met - His - Pro - His - Gly - Ala - Pro - OH (hereafter referred to as "Peptide A").

Rf values: $Rf^1 = 0.03$; $Rf^2 = 0.27$

Elemental analysis: (as H-Pro-Val-Met-His-Pro-His-Gly-Ala-Pro-OH · $CH_3CO_2H$ · $5H_2O$)

| | C(%) | H(%) | N(%) |
|---|---|---|---|
| Calcd. | 48.38 | 7.11 | 16.67 |
| Found | 48.12 | 7.28 | 16.39 |

Amino acid analysis (analyzed with Hitachi 835 Model)

| Amino acid | Analytical data |
|---|---|
| Gly (1) | 1.04 |
| Ala (1) | 1.07 |
| Val (1) | 0.99 |
| Met (1) | 1.00 |
| His (2) | 2.00 |
| Pro (3) | 2.88 |

Synthesis Example 2

In a manner similar to Synthesis Example 1-(2) described above, 1.1 g of the H - Pro - Val - Met - His(Tos) - Pro - His(Tos) - Gly - Ala - Pro - resin and 1.06 g of Boc - Tyr(Cl$_2$ - Bzl) - OH were reacted by double coupling. Then, the removal of the protective groups and the resin was carried out in a manner similar to Synthesis Example 1-(3) described above. The system was similarly purified to obtain 183 mg of H - Tyr - Pro - Val - Met - His - Pro - His - Gly - Ala - Pro - OH (hereafter referred to as "Peptide B").

Rf values: Rf$^1$=0.04: Rf$^2$=0.29

Elemental analysis: (as $C_{51}H_{72}I_{12}N_{14}S \cdot CH_3COOH \cdot 6H_2O$)

|  | C(%) | H(%) | N(%) |
|---|---|---|---|
| Calcd. | 50.03 | 6.96 | 15.40 |
| Found | 49.68 | 7.07 | 15.92 |

Amino acid analysis (analyzed with Hitachi 835 Model)

|  | | Analytical data |
|---|---|---|
| Gly | (1) | 1.07 |
| Ala | (1) | 1.02 |
| Val | (1) | 1.04 |
| Met | (1) | 1.02 |
| Tyr | (1) | 1.02 |
| His | (2) | 2.04 |
| Pro | (3) | 2.80 |

Synthesis Example 3

(1) In 14 ml of a DMSO solution of 5.88 milliequivalents of potassium tert-butoxide 1.54 g of Boc-Leu-OH was dissolved and 5 g of chloromethylated polystyrene resin (Protein Research Promotion Foundation) was added to the solution. The mixture was reacted at 80°C for 30 minutes. After thoroughly washing the resin, in sequence, with DMSO, 50% acetic acid/chloroform and methylene chloride, the resin was dried under reduced pressure to obtain 5.06 g of Boc-Leu-resin.

A part of the Boc-Leu-resin was hydrolyzed and subjected to amino acid analysis. The results indicate that the product contained 0.30 mmol of the amino acid/g of the resin.

(2) After washing 2.17 g of the Boc-Leu-resin obtained in (1) above three times with 30 ml of chloroform, the resin was added to 30 ml of chloroform solution of 50% trifluoroacetic acid (TFA) and the mixture was reacted at room temperature for 20 minutes. The reaction mixture was washed once with 30 ml of chloroform, 5 times with 30 ml of methylene chloride, 3 times with 30 ml of a methylene chloride solution of 10% triethyl amine and then 6 times with 30 ml of methylene chloride to obtain H-Leu-resin.

To 25 ml of a solution of 0.35 g of Boc-Val-OH in methylene chloride the H-Leu-resin described above was added and 5 ml of a solution of 0.33 g of DCC in methylene chloride was then added to the resulting mixture. The mixture was reacted at room temperature for 2 hours. After washing the resin 6 times with 30 ml of methylene chloride, the resin was added to 25 ml of a methylene chloride solution of 0.35 g of Boc-Val-OH and 0.55 g of 1 - hydroxybenzotriazole. Then, 5 ml of a methylene chloride solution of 0.33 g of DCC was added thereto and the resulting mixture was again reacted in a similar manner (double coupling). The resin was thoroughly washed with methylene chloride to obtain Boc-Val-Leu-resin.

(3) In a manner similar to (2) described above, des-Boc of the Boc-Val-Leu-resin was conducted and amino acids described below were then condensed in order, each followed by conducting des-Boc.

| | |
|---|---|
| Boc-Gln-ONP | 0.59 g |
| Boc-Pro-OH | 0.35 g |
| Boc-Ala-OH | 0.31 g |
| Boc-Thr(Bzl)-OH | 0.50 g |
| Boc-Pro-OH | 0.35 g |
| Boc-Glu(OBzl)-OH | 0.55 g |
| Boc-Val-OH | 0.35 g |
| Boc-Tyr(Cl$_2$-Bzl)-OH | 0.71 g |

Thus 2.65 g of H - Tyr(Cl$_2$ - Bzl) - Val - Glu(OBzl) - Pro - Thr(Bzl) - Ala - Pro - Gln - Val - Leu - resin, 1.35 g of which was dissolved in 3 ml of anisole and 30 ml of hydrogen fluoride. After reacting the mixture at −20°C for 30 minutes and then at 0°C for 30 minutes, hydrogen fluoride was removed by distillation. The residue was extracted with 10% acetic acid and the extract was washed with ether. The aqueous layer was freeze dried and then purified by gel filtration using Sephadex G-10 (manufactured by Pharmacia Co., Ltd.; eluting liquid, 10% aqueous acetic acid solution), partition chromatography using Sephadex G-25 (manufactured by Pharmacia Co., Ltd.; eluting liquid, BuOH:AcOH:H$_2$O=4:1:5) and further with LH-20

12

(manufactured by Pharmacia Co., Ltd.; eluting liquid, 1/1000 N-HCl) to obtain H - Tyr - Val - Glu - Pro - Thr - Ala - Pro - Gln - Val - Leu - OH (hereafter referred to as "Peptide C").

Rf values: $Rf^1=0.12$; $Rf^2=0.58$

Elemental analysis: (as $C_{52}H_{81}O_{16}N_{11} \cdot 7H_2O$)

|  | C(%) | H(%) | N(%) |
|---|---|---|---|
| Calcd. | 50.27 | 7.71 | 12.40 |
| Found | 50.41 | 7.83 | 12.41 |

Amino acid analysis (analyzed with Hitachi 835 Model)

|  |  | Analytical data |
|---|---|---|
| Ala | (1) | 1.01 |
| Gl | (1) | 2.09* |
| Glu | (1) |  |
| Leu | (1) | 0.99 |
| Pro | (2) | 2.04 |
| Thr | (1) | 1.04 |
| Tyr | (1) | 0.98 |
| Val | (2) | 1.84 |

*Detected as Glu

Synthesis Example 4

Each of amino acids described below were condensed, in sequence, with 1.70 g of the Boc-Val-resin (0.296 mmol/g resin) obtained in a manner similar to Synthesis Example 3-(1) followed by des-Boc.

| | |
|---|---|
| Boc-Glu(OBzl)-OH | 0.43 g |
| Boc-Gly-OH | 0.22 g |
| Boc-Gly-OH | 0.22 g |
| Boc-Ile-OH(1/2H₂O) | 0.31 g |
| Boc-Ser(Bzl)-OH | 0.38 g |
| Boc-Asn-ONP | 0.46 g |
| Boc-Lys(Cl-Z)-OH | 0.53 g |
| Boc-Pro-OH | 0.28 g |
| Boc-His(Tos)-OH | 0.52 g |
| Boc-Pro-OH | 0.28 g |
| Boc-Ile-OH(1/2H₂O) | 0.31 g |

Thus 2.25 g of H - Ile - Pro - His(Tos) - Pro - Lys(Cl - Z) - Asn - Ser(Bzl) - Ile - Gly - Gly - Glu(OBzl) - Val - resin was obtained, 0.81 g of which was mixed with 15 ml of hydrogen fluoride and 1.5 ml of anisole. The mixture was reacted at −20°C for 30 minutes and then at 0°C for 30 minutes. After removing an excess of hydrogen fluoride by distillation, the residue was extracted with 10% acetic acid. After washing the extract with ether, the system was freeze dried. Then, purification was conducted by gel filtration with Sephadex G-25 (1M acetic acid) and then using CM-Cellulose 23 (manufactured by Whatmann Co., Ltd., 0.04M AcONH₄, pH=7.2) to obtain H - Ile - Pro - His - Pro - Lys - Asn - Ser - Ile - Gly - Gly - Glu - Val - OH (hereafter referred to as "Peptide D").

Rf values: $Rf^1=0.01$; $Rf^2=0.42$

Elemental analysis: (as $C_{55}H_{90}O_{17}N_{16} \cdot 8H_2O$)

|  | C(%) | H(%) | N(%) |
|---|---|---|---|
| Calcd. | 47.47 | 7.68 | 16.10 |
| Found | 47.39 | 7.71 | 15.98 |

Amino acid analysis (analyzed with Hitachi 835 Model)

|  |  | Analytical data |
| --- | --- | --- |
| Asn | (1) | 0.90* |
| Gly | (2) | 1.99 |
| His | (1) | 1.02 |
| Ile | (2) | 2.02 |
| Lys | (1) | 1.01 |
| Pro | (2) | 1.99 |
| Ser | (1) | 0.91 |
| Val | (1) | 1.01 |
| Glu | (1) | 1.02 |

*Detected as Asp

Synthesis Example 5

In a manner similar to Synthesis Example 3-(2), 0.19 g of Boc - Tyr(Cl$_2$ - Bzl) - OH was reacted with 0.77 g of H - Ile - Pro - His(Tos) - Pro - Lys(Cl - Z) - Asn - Ser(Bzl) - Ile - Gly - Gly - Glu(OBzl) - Val - resin,obtained in the same manner as in Synthesis Example 4 by double coupling. Then, the Boc group was split off with TFA to obtain 0.82 g of H - Tyr(Cl$_2$ - Bzl) - Ile - Pro - His(Tos) - Pro - Lys(Cl - Z) - Asn - Ser(Bzl) - Ile - Gly - Gly - Glu(OBzl) - Val - resin. The thus obtained resin was mixed with 15 ml of hydrogen fluoride and 1.5 ml of anisole. The mixture was reacted at −20°C for 30 minutes and then at 0°C for 30 minutes. After removing an excess of hydrogen fluoride by distillation, the residue was extracted with 10% acetic acid. After washing the extract with ether, the system was freeze dried. Then, purification was conducted by gel filtration with Sephadex G-25 (1M acetic acid) and then using CM-Sephadex G-25 (0.04M AcONH$_4$, pH=7.2) to obtain H - Tyr - Ile - Pro - His - Pro - Lys - Asn - Ser - Ile - Gly - Gly - Glu - Val - OH (hereafter referred to as "Peptide E").

Rf values: Rf$^1$=0.02; Rf$^2$=0.47

Elemental analysis: (as C$_{64}$H$_{99}$O$_{19}$N$_{17}$ · 8H$_2$O)

|  | C(%) | H(%) | N(%) |
| --- | --- | --- | --- |
| Calcd. | 49.44 | 7.46 | 15.32 |
| Found | 49.43 | 7.56 | 15.06 |

Amino acid analysis (analyzed with Hitachi 835 Model)

|  |  | Analytical data |
| --- | --- | --- |
| Asn | (1) | 0.89* |
| Gly | (2) | 1.99 |
| Glu | (1) | 1.02 |
| His | (1) | 1.02 |
| Ile | (2) | 2.04 |
| Lys | (1) | 1.02 |
| Pro | (2) | 2.32 |
| Ser | (1) | 0.91 |
| Val | (1) | 1.01 |
| Tyr | (1) | 1.05 |

*Detected as Asp

Synthesis Example 6

In a manner similar to Synthesis Example 3-(1), Boc-Leu-resin was prepared. Each of amino acids shown below was reacted, in sequence, with 2.17 g of the thus obtained resin by double coupling in a manner similar to Synthesis Example 3-(2) and (3) described above. The des-Boc reaction of the Trp-containing peptide was carried out in the presence of ethanedithiol.

| | |
|---|---|
| Boc-Val-OH | 0.35 g |
| Boc-Lys(Cl-Z)-OH | 0.66 g |
| Boc-Thr(Bzl)-OH | 0.50 g |
| Boc-Lys(Cl-Z)-OH | 0.66 g |
| Boc-Asp(OBzl)-OH | 0.52 g |
| Boc-Lys(Cl-Z)-OH | 0.66 g |
| Boc-Pro-OH | 0.35 g |
| Boc-Thr(Bzl)-OH | 0.50 g |
| Boc-Trp-OH | 0.49 g |
| Boc-Thr(Bzl)-OH | 0.50 g |

Thus 3.01 g of H - Thr(Bzl) - Trp - Thr(Bzl) - Pro - Lys(Cl - Z) - Asp(OBzl) - Lys(Cl - Z) - Thr(Bzl) - Lys(Cl - Z) - Val - Leu - resin was obtained. In 15 ml of hydrogen fluoride, 1.5 ml of anisole and 0.8 ml of ethanedithiol 1.10 g of the thus obtained resin was dissolved. The solution was reacted at −20°C for 30 minutes and then at 0°C for 30 minutes. After removing an excess of hydrogen fluoride by distillation, the residue was extracted with 10% acetic acid. After washing the extract with ether, the system was freeze dried. Then, purification was conducted by gel filtration with Sephadex G-25 (1M acetic acid), CM-Cellulose 23 (0.05M AcONH$_4$, pH=7.2) and LH-20 (10$^{-3}$ NHCl) to obtain H - Thr - Trp - Thr - Pro - Lys - Asp - Lys - Thr - Lys - Val - Leu - OH (hereafter referred to as "Peptide F").

Rf values: Rf$^1$=0.01; Rf$^2$=0.44

Elemental analysis: (as C$_{81}$H$_{101}$O$_{17}$N$_{15}$ · 17H$_2$O)

| | C(%) | H(%) | N(%) |
|---|---|---|---|
| Calcd. | 45.15 | 8.39 | 12.95 |
| Found | 45.12 | 8.65 | 12.89 |

Amino acid analysis (analyzed with Hitachi 835 Model)

| | | Analytical data |
|---|---|---|
| Asp | (1) | 0.91 |
| Lys | (3) | 3.20 |
| Leu | (1) | 1.00 |
| Pro | (1) | 1.00 |
| Thr | (3) | 2.89 |
| Trp | (1) | 0.93 |
| Val | (1) | 0.98 |

Synthesis Example 7

Boc - Tyr(Cl$_2$ - Bzl) - OH, 0.13 g was reacted with 0.55 g of H - Thr(Bzl) - Trp - Thr(Bzl) - Pro - Lys(Cl - Z) - Asp(OBzl) - Lys(Cl - Z) - Thr(Bzl) - Lys(Cl - Z) - Val - Leu - resin by double coupling. Then the Boc group was split off with TFA in the presence of ethanedithiol to obtain 0.59 g of H - Thr(Cl$_2$ - Bzl) - Trp - Thr(Bzl) - Pro - Lys(Cl - Z) - Asp(OBzl) - Lys(Cl - Z) - Thr(Bzl) - Lys(Cl - Z) - Val - Leu - resin.

The thus obtained resin was dissolved in 15 ml of hydrogen fluoride, 1.5 ml of anisole and 0.8 ml of ethanedithiol. The solution was reacted at −20°C for 30 minutes and then at 0°C for 30 minutes. After removing an excess of hydrogen fluoride by distillation, the residue was extracted with 10% acetic acid. After washing the extract with ether, the system was freeze dried. Then, purification was conducted with CM-Cellulose 23 (0.05M AcONH$_4$, pH=7.2) and LH-20 (10$^{-3}$ NHCl) to obtain H - Tyr - Thr - Trp - Thr - Pro - Lys - Asp - Lys - Thr - Lys - Val - Leu - OH (hereafter referred to as "Peptide G").

Rf values: Rf$^1$=0.01; Rf$^2$=0.47

Elemental analysis: (as C$_{70}$H$_{110}$O$_{19}$N$_{16}$ · 17H$_2$O)

| | C(%) | H(%) | N(%) |
|---|---|---|---|
| Calcd. | 47.08 | 8.13 | 12.55 |
| Found | 46.89 | 8.34 | 12.54 |

Amino acid analysis (analyzed with Hitachi 835 Model)

|  |  | Analytical data |
|---|---|---|
| Asp | (1) | 0.91 |
| Lys | (3) | 3.10 |
| Leu | (1) | 1.02 |
| Pro | (1) | 1.20 |
| Thr | (3) | 2.85 |
| Trp | (1) | 0.92 |
| Val | (1) | 0.93 |

Synthesis Example 8

(1) In 42 ml of a DMSO solution of 15.33 milliequivalents of potassium tert-butoxide 7.53 g of Boc-Tyr(Cl$_2$-Bzl)-OH was dissolved and 10 g of chloromethylated polystyrene resin (Protein Research Promotion Foundation) was added to the solution. The mixture was reacted at 80°C for 30 minutes. after thoroughly washing the resin, in sequence, with DMSO, 50% acetic acid/chloroform and methylene chloride, the resin was dried under reduced pressure to obtain 12 g of Boc-Tyr(Cl$_2$-Bzl)-resin.

A part of the thus obtained resin was hydrolyzed and subjected to amino acid analysis. The results indicate that the product contained 0.31 mmol of the amino acid/g of the resin.

(2) After washing 1.70 g of the Boc-Tyr(Cl$_2$-Bzl)-resin obtained in (1) above three times with 30 ml of chloroform, the resin was added to 30 ml of a chloroform solution of 50% trifluoroacetic acid (TFA) and the mixture was reacted at room temperature for 20 minutes. The reaction mixture was washed once with 30 ml of chloroform, 5 times with 30 ml of methylene chloride, 3 times with 30 ml of a methylene chloride solution of 10% triethyl amine and then 6 times with 30 ml of methylene chloride, to obtain H-Tyr(Cl$_2$-Bzl)-resin.

To 25 ml of a solution of 0.28 g of Boc-Pro-OH in methylene chloride the H-Tyr(Cl$_2$-Bzl)-resin described above was added and 5 ml of a solution of 0.27 g of DCC in methylene chloride was then added to the resulting mixture. The mixture was reacted at room temperature for 2 hours. After washing the resin 6 times with 30 ml of methylene chloride, the resin was added to 25 ml of a methylene chloride solution of 0.28 g of Boc-Pro-OH and 0.55 g of 1 - hydroxybenzotriazole. Then, 5 ml of a methylene chloride solution of 0.27 g of DCC was added thereto and the resulting mixture was again reacted in a similar manner (double coupling). The resin was thoroughly washed with methylene chloride to obtain Boc-Pro-Tyr(Cl$_2$-Bzl)-resin.

(3) In a manner similar to (2) described above, des-Boc of the Boc-Pro-Tyr(Cl$_2$-Bzl)-resin was conducted. Then, amino acids, amino acids wherein functional groups at the side chain thereof were protected or carboxyl groups were activated, described below were condensed in order, followed by conducting des-Boc.

| Boc-Pro-OH | 0.28 g |
|---|---|
| Boc-Pro-OH | 0.28 g |
| Boc-Lys(Cl-Z)-OH | 0.55 g |
| Boc-Lys(Cl-Z)-OH | 0.55 g |
| Boc-Pro-OH | 0.28 g |
| Boc-Gln-ONP | 0.48 g |
| Boc-Val-OH | 0.29 g |
| Boc-Val-OH | 0.29 g |

Thus 2.57 g of H - Val - Val - Gln - Pro - Lys(Cl - Z) - Lys(Cl - Z) - Pro - Pro - Pro - Tyr(Cl$_2$ - Bzl) - resin. The thus obtained resin, 1.20 g, was dissolved in 2 ml of anisole and 20 ml of hydrogen fluoride. The solution was incubated at −20°C for 30 minutes and then at 0°C for 30 minutes. Thereafter an excess of hydrogen fluoride was removed by distillation. The residue was extracted with 10% acetic acid and the extract was washed with ether. The aqueous layer was freeze dried and then purified by gel filtration using Sephadex G-25 (manufactured by Pharmacia Co., Ltd.; eluting liquid, 1M acetic acid) and further using CM-23 Cellulose (manufactured by Whatmann Co., Ltd.; eluting liquid, 0.04M ammonium acetate; pH=7.2) to obtain 162 mg of H - Val - Val - Gln - Pro - Lys - Lys - Pro - Pro - Pro - Tyr - OH (hereafter referred to as "Peptide H").

Rf values: Rf[1]=0.01; Rf[2]=0.27

Elemental analysis: (as C$_{56}$H$_{89}$O$_{13}$N$_{13}$ · 3CH$_3$CO$_2$H · 4H$_2$O)

|  | C(%) | H(%) | N(%) |
|---|---|---|---|
| Calcd. | 53.02 | 7.82 | 12.96 |
| Found | 52.94 | 8.06 | 12.74 |

Amino acid analysis (analyzed with Hitachi 835 Model)

|          |     | Analytical data |
| -------- | --- | --------------- |
| Gln      | (1) | 1.05*           |
| Lys      | (2) | 2.17            |
| Pro      | (4) | 4.02            |
| Tyr      | (1) | 1.05            |
| Val      | (2) | 1.69            |

*Detected as Glu

Synthesis Example 9

In a manner similar to Synthesis Example 8-(1), Boc-Pro-resin (0.44 mmol/g resin) was prepared. Each of amino acids or derivatives thereof shown below was reacted, in sequence, with 1.20 g of the thus obtained resin by double coupling in a manner similar to Synthesis Example 8-(2) and (3) described above. The des-Boc reaction was then carried out.

|                              |         |
| ---------------------------- | ------- |
| Boc-Ser(Bz)-OH               | 0.39 g  |
| Boc-Ala-OH                   | 0.25 g  |
| Boc-Ser(Bzl)-OH              | 0.39 g  |
| Boc-Arg(Tos)-OH              | 0.56 g  |
| Boc-Ser(Bzl)-OH              | 0.39 g  |
| Boc-Phe-OH                   | 0.35 g  |
| Boc-Ile-OH 1/2H$_2$O         | 0.32 g  |
| Boc-Gln-ONP                  | 0.49 g  |
| Boc-Gly-OH                   | 0.23 g  |
| Boc-Met-OH                   | 0.33 g  |

Thus 1.58 g of H - Met - Gly - Gln - Ile - Phe - Ser(Bzl) - Arg(Tos) - Ser(Bzl) - Ala - Ser(Bzl) - Pro - resin was obtained. In 10 ml of hydrogen fluoride, 0.5 ml of 1,2 - ethanedithiol and 1 ml of anisole 0.40 g of the thus obtained resin was dissolved. The solution was incubated at −20°C for 30 minutes and then at 0°C for 30 minutes. After removing an excess of hydrogen fluoride by distillation under reduced pressure, the residue was extracted with 10% acetic acid. After washing the extract with ether, the system was freeze dried.

Then, purification was conducted by gel filtration with Sephadex G-25 (manufactured by Pharmacia Co., Ltd.; eluting liquid, 1M acetic acid) and CM-Cellulose 23 (manufactured by Whatmann Co., Ltd.; 0.1 to 0.5M ammonium acetate, pH=5.0, linear concentration gradient) to obtain 32 mg of H - Met - Gly - Gln - Ile - Phe - Ser - Arg - Ser - Ala - Ser - Pro - OH (hereafter referred to as "Peptide I").

Rf values: Rf$^1$=0.01; Rf$^2$=0.50

Elemental analysis: (as C$_{50}$H$_{81}$O$_{16}$N$_{15}$S · CH$_3$CO$_2$H · 5H$_2$O)

|        | C(%)  | H(%)  | N(%)  |
| ------ | ----- | ----- | ----- |
| Calcd. | 46.94 | 7.20  | 15.79 |
| Found  | 47.01 | 7.32  | 15.66 |

Amino acid analysis (analyzed with Hitachi 835 Model)

|          |     | Analytical data |
| -------- | --- | --------------- |
| Ala      | (1) | 1.07            |
| Arg      | (1) | 1.07            |
| Gln      | (1) | 1.00*           |
| Gly      | (1) | 1.00            |
| Ile      | (1) | 0.91            |
| Met      | (1) | 0.94            |
| Phe      | (1) | 0.93            |
| Pro      | (1) | 1.07            |
| Ser      | (3) | 2.98            |

*Detected as Glu

Synthesis Example 10

In a manner similar to Synthesis Example 8-(2) described above, 0.47 g of Boc-Tyr(Cl$_2$-Bzl)-OH was

reacted with 0.41 g of H - Met - Gly - Gln - Ile - Phe - Ser(Bzl) - Arg(Tos) - Ser(Bzl) - Ala - Ser(Bzl) - Pro - resin by double coupling. Then, the Boc group was split off with trifluoroacetic acid to obtain H - Tyr(Cl$_2$ - Bzl) - Met - Gly - Gln - Ile - Phe - Ser(Bzl) - Arg(Tos) - Ser(Bzl) - Ala - Ser(Bzl) - Pro - resin. The thus obtained resin was mixed with 10 ml of hydrogen fluoride, 1 ml of anisole and 0.5 ml of 1,2 - ethanedithiol. The mixture was incubated at −20°C for 30 minutes and then at 0°C for 30 minutes. After removing an excess of hydrogen fluoride by distillation under reduced pressure, the residue was extracted with 10% acetic acid. After washing the extract with ether, the system was freeze dried. Then, purification was conducted by gel filtration with Sephadex G-25 (manufactured by Pharmacia Co., Ltd., 1M acetic acid) and then by HPLC using a debelogil column (manufactured by Chemco Co., Ltd.; eluting liquid, 0.1M NaH$_2$PO$_4$:acetonitrile=80:20). Thereafter, the purified matter was desalted with Sephadex G-25 (manufactured by Pharmacia Co., Ltd.; eluting liquid, 1M acetic acid) to obtain 9.23 mg of H - Tyr - Met - Gly - Gln - Ile - Phe - Ser - Arg - Ser - Ala - Ser - Pro - OH (hereafter referred to as "Peptide J").

Rf values: Rf$^1$=0.01; Rf$^2$=0.52

Elemental analysis: (as C$_{59}$H$_{90}$O$_{18}$N$_{16}$S · CH$_3$CO$_2$H · 6H$_2$O)

|  | C(%) | H(%) | N(%) |
|---|---|---|---|
| Calcd. | 48.47 | 7.07 | 14.82 |
| Found | 48.63 | 7.01 | 14.77 |

Amino acid analysis (analyzed with Hitachi 835 Model)

|  |  | Analytical data |
|---|---|---|
| Ala | (1) | 1.03 |
| Met | (1) | 1.00 |
| Arg | (1) | 1.07 |
| Phe | (1) | 1.09 |
| Gln | (1) | 1.03* |
| Pro | (1) | 0.99 |
| Gly | (1) | 1.14 |
| Ser | (3) | 2.68 |
| Ile | (1) | 1.03 |
| Tyr | (1) | 1.03 |

*Detected as Glu

Synthesis Example 11

In a manner similar to Example 8-(1), 0.27 mmol/g resin of Boc-Leu-resin was prepared. Each of amino acids or derivatives thereof described below were reacted, in sequence, with 2 g of the Boc-Leu-resin by double coupling in a manner similar to Synthesis Example 6-(2) and (3). Then des-Boc reaction followed.

| Boc-Ser-OH | 0.40 g |
|---|---|
| Boc-Arg(Tos)-OH | 0.58 g |
| Boc-Leu-OH · H$_2$O | 0.34 g |
| Boc-Ile-OH · 1/2H$_2$O | 0.32 g |
| Boc-Pro-OH | 0.29 g |
| Boc-Asp(OBzl)-OH | 0.44 g |
| Boc-Lys(Cl-Z)-OH | 0.56 g |
| Boc-Pro-OH | 0.29 g |
| Boc-Thr(Bzl)-OH | 0.39 g |
| Boc-Gly-OH | 0.24 g |
| Boc-Glu(OBzl)-OH | 0.46 g |
| Boc-Pro-OH | 0.29 g |
| Boc-Tyr(Cl$_2$-Bzl)-OH | 0.57 g |

Thus 2.62 g of Boc - Tyr(Cl$_2$ - Bzl) - Pro - Glu(OBzl) - Gly - Thr(Bzl) - Pro - Lys(Cl - Z) - Asp(OBzl) - Pro - Ile - Leu - Arg(Tos) - Ser - Leu - resin was obtained. In 20 ml of hydrogen fluoride and 2 ml of anisole 1.62 g of the thus obtained resin was dissolved. The solution was reacted at −20°C for 30 minutes and then at 0°C for 30 minutes. After removing an excess of hydrogen fluoride by distillation under reduced pressure, the residue was extracted with 10% acetic acid. After washing the extract with ether, the system was freeze dried. Then, the extract with ether, the system was freeze dried. Then, purification was conducted by gel filtration with Sephadex G-25 (1M acetic acid) and then HPLC using debelogil column (eluting liquid, 0.1M phosphite buffer:acetonitrile=80:20) to obtain 104 mg of a peptide, H - Tyr - Pro - Glu - Gly - Thr - Pro - Lys - Asp - Pro - Ile - Leu - Arg - Ser - Leu - OH (hereafter referred to as "Peptide K").

Rf values: $Rf^1 = 0.01$; $Rf^2 = 0.42$

Elemental analysis: (as $C_{72}H_{116}O_{22}N_{18} \cdot CH_3CO_2H \cdot 7H_2O$)

|  | C(%) | H(%) | N(%) |
|---|---|---|---|
| Calcd. | 50.16 | 7.62 | 14.23 |
| Found | 49.87 | 7.60 | 14.29 |

Amino acid analysis (analyzed with Hitachi 835 Model)

|  |  | Analytical data |
|---|---|---|
| Asp | (1) | 0.96 |
| Lys | (1) | 1.13 |
| Arg | (1) | 1.02 |
| Pro | (3) | 2.79 |
| Glu | (1) | 1.05 |
| Ser | (1) | 0.92 |
| Gly | (1) | 1.03 |
| Thr | (1) | 0.99 |
| Ile | (1) | 0.99 |
| Tyr | (1) | 0.97 |
| Leu | (2) | 2.11 |

Synthesis Example 12

The same procedures as in Synthesis Example 11 were repeated except that 0.58 g of Boc-Ser(Bzl)-OH was used in place of 0.40 g of Boc-Ser-OH to obtain 120 mg of Peptide K having the same physical properties as above.

Preparation of antigen

Preparation Example 1

To 3.0 ml of a 0.05M phosphate buffer (pH=7.0) 5 mg of Peptide A obtained in Synthesis Example 1 and 25 mg of a protein extracted from the ascaris (ASC) were added and 0.2 ml of a 2% glutaraldehyde (GA) solution was dropwise added to the resulting solution. The mixture was stirred at room temperature for 3 hours. Thereafter the reaction mixture was dialyzed at 4°C with distilled water overnight. After freeze drying, 29 mg of an immune antigen was obtained. The antigen is hereafter referred to as "Antigen A").

Antigen A bound 10 mols in average of Peptide A per 1 ml of the ascaris (when an average molecular weight was made 100,000; the same is true hereinafter). This binding rate of Peptide A to the ascaris was determined as follows: A fraction of Peptide A bound to the ascaris was separated from another fraction of other product (dimer of Peptide A) by gel filtration of Antigen A obtained wit Sephadex G-50 (eluting liquid, physiological saline solution; detection, OD 280 nm; eluting rate, 3 ml/hour; fractionated amount, 1 ml each); a calibration curve of a peptide dimer having standard concentrations was prepared to determine the amount of the aforesaid dimer; and the thus determined amount of the dimer was subtracted from the amount of Peptide A used as a raw material, assuming that the thus subtracted amount would be all bound to the ascaris since neither the unreacted ascaris nor Peptide A was recognized. Such is hereafter the same also in the following examples for preparing antigens.

Preparation Example 2

To 3.0 ml of a 0.05M phosphate buffer (pH=7.0) 5 mg of Peptide A obtained in Synthesis Example 1 and 25 mg of a protein extracted from the ascaris were added and 200 mg of dicyclohexylcarbodiimide (DCC) was dropwise added to the resulting solution. The mixture was stirred at room temperature for 3 hours. Thereafter the reaction mixture was dialyzed at 4°C with distilled water overnight. After freeze drying, 28 mg of an immune antigen was obtained. The antigen is hereafter referred to as "Antigen B".

Antigen B bound thereto 12 mols of Peptide A per 1 mol of the ascaris in average.

Preparation Example 3

An immune antigen was obtained in a manner similar to Preparation Example 1 described above except that KLH (Sigma Co., Ltd.) was used instead of the protein extracted from the ascaris. Hereafter the antigen is referred to as "Antigen C". Antigen C bound thereto 10 mols of Peptide A per 1 mlol of KLH in average (when an average molecular weight was made 100,000; the same is true hereinafter).

Preparation Example 4

An immune antigen was obtained in a manner similar to Preparation Example 2 described above except that KLH (Sigma Co., Ltd.) was used instead of the protein extracted from the ascaris. Hereafter the antigen is referred to as "Antigen D". Antigen D bound thereto 9 mols of Peptide A per 1 mol of KLH in average.

Preparation Example 5

An immune antigen was obtained in a manner similar to Preparation Example 2 described above except that BSA and Peptide B were used instead of the protein extracted from the ascaris and Peptide A, respectively. Hereafter the antigen is referred to as "Antigen E". Antigen E bound thereto 15 mols of Peptide B per 1 mol of BSA in average.

Preparation Example 6

To 3.0 ml of a 0.05M phosphate buffer (pH=7.0) 5 mg of Peptide C obtained in Synthesis Example 3 and 12 mg of KLH (Sigma Co., Ltd.) were added and 0.2 ml of a 2% glutaraldehyde solution was dropwise added to the resulting solution. The mixture was stirred at room temperature for 3 hours. Thereafter the reaction mixture was dialyzed at 4°C with distilled water overnight. After freeze drying, 16.5 mg of the desired antigen was obtained. The antigen is hereafter referred to as "Antigen F".

Antigen F bound thereto 10 mols of Peptide C per 1 mol of KLH (when an average molecular weight was made 100,000) in average.

Preparation Example 7

(1) a BDB solution was prepared by adding 83.25 mg of benzidine to a solvent mixture of 20 ml of 0.2N-HCl and 3 ml of DMF, stirring the mixture under ice cooling, gradually adding 2 ml of distilled water containing 87.03 mg of sodium nitrite to the solution and then stirring the mixture for 30 minutes.

(2) In 1 ml of a 0.16M borate buffer (pH=9.0) containing 0.13M NaCl were dissolved in 5.08 mg of Peptide G and 8.07 mg of KLH. The solution was slowly stirred at 4°C. To the solution 1 ml of the BDB solution obtained in (1) above was gradually added dropwise. The reaction solution was adjusted with 0.5N NaOH to pH=9.0 followed by reacting for further 2 hours at 4°C. Thereafter the reaction mixture was dialyzed at 4°C with distilled water overnight. After freeze drying, 12.27 mg of the desired antigen was obtained. Hereafter the antigen is referred to as "Antigen G". Antigen G bound thereto 35 mols of Peptide G per 1 mol of KLH in average.

Preparation Example 8

In a manner similar to Preparation Example 7 described above, 12.74 mg of the desired antigen was obtained except for using 5.17 mg of Peptide E and 8.03 mg of KLH. Hereafter the antigen is referred to as "Antigen H". Antigen H bound thereto 42 mols of Peptide E per 1 mol of KLH in average.

Preparation Example 9

Following the preparation examples described above, immune antigens shown in Table 2 below were obtained.

TABLE 2

| Antigen | Hapten (mg) | Carrier (mg) | Binding agent (mg) | Molar ratio of carrier-hapten bond |
|---|---|---|---|---|
| I | Peptide C (3) | ASC (6) | GA (0.59) | 1:24 |
| J | Peptide G (2) | ASC (4) | BDB (0.75) | 1:14 |
| K | Peptide C (3) | ASC (6) | BDB (1.50) | 1:18 |
| L | Peptide D (3) | ASC (6) | DCC (0.67) | 1:25 |
| M | Peptide E (3) | ASC (6) | BDB (1.19) | 1:22 |
| N | Peptide F (3) | ASC (6) | DCC (0.50) | 1:16 |

Preparation Example 10

To 3.0 ml of a 0.05M phosphate buffer (pH=7.0) 5.09 mg of Peptide I obtained in Synthesis Example 9 and 25.10 mg of KLH were added and 0.2 ml of a 2% glutaraldehyde solution was dropwise added to the resulting solution. The mixture was stirred at room temperature for 3 hours. Thereafter the reaction mixture was dialyzed at 4°C with distilled water overnight. After freeze drying, 26.49 mg of the desired antigen was obtained. The antigen is hereafter referred to as "Antigen O".

Antigen O bound thereto 10 mols of Peptide I per 1 mol of KLH (when an average molecular weight was made 100,000) in average.

Preparation Example 11

(1) A BDB solution was prepared by adding 83.25 mg of benzidine to a solvent mixture of 20 ml of 0.2N-HCl and 3 ml of DMF, stirring the mixture under ice cooling, gradually adding 2 ml of distilled water containing 87.03 mg of sodium nitrite to the solution and then stirring the mixture for 30 minutes.

(2) In 1 ml of a 0.16M borate buffer (pH=9.0) containing 0.13M NaCl were dissolved 5.13 mg of Peptide K and 8.10 mg of KLH. The solution was slowly stirred at 4°C. To the solution 1 ml of the BDB solution obtained in (1) above was gradually added dropwise. The reaction solution was adjusted with 0.5N NaOH to pH=9.0 followed by reacting for further 2 hours at 4°C. Thereafter the reaction mixture was dialyzed at 4°C with distilled water overnight. After freeze drying, 12.76 mg of the desired antigen was obtained. Hereafter the antigen is referred to as "Antigen P". Antigen P bound thereto 18 mols of Peptide K per 1 mol of KLH in average.

Preparation Example 12

In a manner similar to Preparation Example 11 described above, 12.74 mg of the desired antigen was obtained except for using 5.17 mg of Peptide H and 8.03 mg of KLH. Hereafter the antigen is referred to as "Antigen Q". Antigen Q bound thereto 25 mols of Peptide H per 1 mol of KLH in average.

Preparation Example 13

Following the preparation examples described above, immune antigens shown in Table 3 below were obtained.

TABLE 3

| Antigen | Hapten (mg) | Carrier (mg) | Binding agent (mg) | Molar ratio of carrier-hapten bond |
|---------|-------------|--------------|--------------------|-----------------------------------|
| R | Peptide I (2) | ASC (4) | GA (0.37) | 1:19 |
| S | Peptide I (2) | ASC (4) | DCC (0.70) | 1:21 |
| T | Peptide K (2) | ASC (4) | DCC (0.57) | 1:17 |
| U | Peptide K (2) | ASC (4) | GA (0.30) | 1:14 |
| V | Peptide K (2) | ASC (4) | BDB (0.69) | 1:13 |
| W | Peptide H (5) | ASC (10) | BDB (2.42) | 1:19 |
| X | Peptide J (3) | BSA (6) | DCC (0.40) | 1:22 |

Preparation of antibody

Preparation Example 14

(1) After dissolving 100 μg of Antigen A obtained in Preparation Example (of Antigen) 1 in 1.5 ml of a physiological saline solution, respectively, 1.5 ml of a Freund's adjuvant was added to the solution to obtain a suspension. The suspension was subcutaneously administered to 3 rabbits (2.5 to 3.0 kg). The suspension was given at the same dose 9 times every 2 weeks. After the final administration, blood was collected from the test animals. Anti sera (ATLA antibodies of the present invention) were obtained by centrifugation. The antibodies are referred to Antibody A, Antibody B and Antibody C, respectively, to each of the rabbits.

(2) In a manner similar to Preparation Example (of Antibody) 14-(1) above, ATLA antibodies of the present invention were obtained from 6 rabbits (2.5 to 3.0 kg) except that Antigen B obtained in Preparation Example (of Antigen) 2 described above was employed. The antibodies are referred to as Antibody D, Antibody E, Antibody F, Antibody G, Antibody H and Antibody I, respectively, to each of the rabbits.

(3) In a similar manner to Preparation Example (of Antibody) 14-(1) above, ATLA antibodies were obtained except that Antigen C, Antigen D and Antigen E obtained in Preparation Examples (of Antigen) 3, 4 and 5, respectively, were employed. The thus obtained antibodies are referred to as Antibody J, Antibody K and Antibody L, respectively.

Preparation Example 15

After dissolving 100 μg of each of Antigens F, G and H obtained in Preparation Examples (of Antigen) 6 to 8 in 1.5 ml of a physiological saline solution, respectively, 1.5 ml of a Freund's adjuvant was added to each of the solutions to obtain suspensions. Each of the suspensions was subcutaneously administered to several rabbits (New Zealand white rabbits), respectively. Each of the suspensions was given at the same dose 6 times every 2 weeks and then at the same dose as the initial dose 3 times monthly. Seven days after the final administration, blood was collected from the test animals. Anti sera were obtained by centrifugation and the desired antibodies were obtained, respectively. Antibodies are referred to as follows: "Antibody M" which was obtained from Antigen F, "Antibody N", "Antibody O", "Antibody P", "Antibody Q" and "Antibody R" from Antigen G; "Antibody S", "Antibody T", "Antibody U", "Antibody V" and "Antibody W" from Antigen H.

Preparation Example 16

Using each 500 μm of the antigens obtained in Preparation Example 9 of Antigen described above, antibodies shown in Table 4 below were obtained, respectively, in a manner similar to Preparation Example 15 of Antibody described above.

TABLE 4

| Antigen No. | Antibody No. |
|---|---|
| Antigen I | Antibody X |
| | Antibody Y |
| | Antibody Z |
| Antigen J | Antibody AA |
| | Antibody AB |
| | Antibody AC |
| Antigen K | Antibody AD |
| | Antibody AE |
| | Antibody AF |
| Antigen L | Antibody AG |
| Antigen M | Antibody AH |
| | Antibody AI |
| | Antibody AJ |
| Antigen N | Antibody AK |

Preparation Example 17

After dissolving 100 μg of each of Antigens O, P and Q obtained in Preparation Examples (of Antigen) 10 to 12 in 1.5 ml of a physiological saline solution, respectively, 1.5 ml of a Freund's adjuvant was added to each of the solutions to obtain suspensions. Each of the suspensions was subcutaneously administered to several rabbits (New Zealand white rabbits) (2.5 to 3.0 kg), respectively. Each of the suspensions was given to the rabbits at the same dose level 6 times every 2 weeks and then at the same dose as the initial dose 3 times monthly. Seven days after the final administration, blood was collected from the test animals. Anti sera were obtained by centrifugation and the desired antibodies were obtained, respectively. Antibodies are referred to as follows: "Antibody AL" which was obtained from Antigen O; "Antibody AM", "Antibody AN", "Antibody AO", "Antibody AP" and "Antibody AQ" from Antigen P; and "Antibody AR" from Antigen Q.

Preparation Example 18

Using each 500 μg of the antigens obtained in Preparation Example 13 of Antigen described above,

22

antibodies shown in Table 5 below were obtained, respectively, in a manner similar to Preparation Example 17 of Antibody described above.

TABLE 5

| Antigen No. | Antibody No. |
| --- | --- |
| Antigen R | Antibody AS |
|  | Antibody AT |
| Antigen S | Antibody AU |
|  | Antibody AV |
| Antigen T | Antibody AW |
|  | Antibody AX |
| Antigen U | Antibody AY |
|  | Antibody AZ |
| Antigen V | Antibody BA |
|  | Antibody BB |
| Antigen W | Antibody BC |
|  | Antibody BD |
|  | Antibody BE |
|  | Antibody BF |
|  | Antibody BG |
| Antigen X | Antibody BH |

Preparation Example 19

. Using 500 µg of Antigen A prepared in Synthesis Example 1 of antigen described above, an antibody was obtained in a manner similar to Preparation Example 14 of Antibody described above. This antibody is referred to as Antibody BI.

Preparation of labelled peptide
Preparation Example 20

Peptide B obtained in Synthesis Example 2 was labelled in accordance with the method using chloramin T as follows:

That is, 20 µl of a 0.5M phosphate buffer containing 1 mCi of Na ($^{125}$I) (carrier free N.E.N.) was added to 10 µl of a 0.5M phosphate buffer (pH 7.5) containing 5 µg of the aforesaid peptide and then 20 µl of a 0.5M phosphate buffer containing 20 µl of chloramin T was added thereto. After stirring the mixture for 25 seconds at room temperature, 20 µl of a 0.5M phosphate buffer containing 100 µg of sodium metabisulfite ($Na_2S_2O_5$) was added to the mixture to complete the reaction. Then, 10 µl of a cold 10% aqueous sodium iodide solution was added to the reaction mixture. The reaction mixture was passed through a Sephadex G-25 column (1.0 to 50 cm) (eluting liquid, a 0.2M ammonium acetate buffer containing 0.1% BSA and 0.01% $NaN_3$; pH 5.5) to obtain Peptide B labelled with $^{125}$I.

The radioactivity of the thus labelled peptide was 255 µCi/µg, which is referred to as "Labelled Peptide B".

Preparation Example 21

In a manner similar to Preparation Example 20 (of Labelled Peptide) described above, each of peptides labelled with $^{125}$I were obtained, respectively, except that Peptide C, Peptide E, Peptide G, Peptide H, Peptide J and Peptide K were employed in lieu of Peptide B, respectively. The radioactivity of each of the peptides was not lower than 1000 µCi/µg.

Measurement of titer

The titer of each of the antibodies obtained as described above was measured as follows:

That is, each of the antibodies was diluted with a physiological saline solution to 10, $10^2$, $10^3$, $10^4$, $10^5$, . . . times, respectively. To 100 µl each of the thus obtained dilutions, were added 0.1 ml of a labelled peptide diluted to about 9500 cpm (in the case of Antibodies A to L, Labelled Peptide B was employed; Labelled Peptide C in the case if Antibodies M, X to Z, AD to AF; Labelled Peptide E in the case of Antibodies S to W and AG to AJ; Labelled Peptide G in the case of Antibodies N to R, AA to AC and AK; Labelled Peptide H in the case of Antibodies AR and BC to BG; Labelled Peptide J in the case of Antibodies AL, AS to AV and BH; and Labelled Peptide K in the case of Antibodies AM to AQ and AW to BB, respectively) and

23

0.2 ml of a 0.05M phosphate buffer (pH=7.4; containing 0.25% BSA, 10 mM EDTA and 0.02% $NaN_3$). The mixture was incubated at 4°C for 24 hours. The resulting antibody[125]I-labelled peptide complex was separated from the unreacted (unbound) [125]I-labelled peptide by the dextran-activated charcoal method and the centrifugal method (4°C, 30 minutes, 3000 rpm) and the radioactive ray was counted to measure a binding rate (%) of the antibody to the [125]I-labelled peptide at each of the dilution concentrations. The binding rate (%) of the antibody to the [125]I-labelled peptide is taken on the vertical axis and the dilution magnification of the antibody is taken on the abscissa. At each of the concentrations, the binding rate is plotted. Then, the dilution magnification of the antibody where the binding rate shows 50%, i.e., a titer of the antibody, is determined.

The results obtained are shown in Table 6 below.

TABLE 6

| Antibody No. | Titer |
| --- | --- |
| A | 50000 |
| B | 1000 |
| C | 1500 |
| D | 30000 |
| E | 2500 |
| F | 20000 |
| G | 3000 |
| H | 15000 |
| I | 5000 |
| J | 6000 |
| K | 3000 |
| L | 8000 |
| M | 5000 |
| N | 250 |
| O | 1500 |
| P | 32000 |
| Q | 3400 |
| R | 5000 |
| S | 4200 |
| T | 720 |
| U | 1800 |
| V | 420 |
| W | 750 |
| X | 62500 |
| Y | 16500 |
| Z | 2300 |
| AA | 7500 |
| AB | 18500 |
| AC | 1800 |
| AD | 11750 |
| AE | 2500 |
| AF | 3250 |
| AG | 3000 |
| AH | 19000 |
| AI | 2500 |
| AJ | 9000 |
| AK | 3500 |
| AL | 1000 |
| AM | 1500 |
| AN | 50 |
| AO | 5000 |
| AP | 2500 |
| AQ | 1000 |
| AR | 7500 |
| AS | 780 |
| AT | 2550 |
| AU | 15000 |
| AV | 2700 |
| AW | 175 |
| AX | 125 |
| AY | 1000 |
| AZ | 2650 |
| BA | 5250 |
| BB | 6000 |
| BC | 2100 |
| BD | 675 |
| BE | 9500 |
| BF | 70000 |
| BG | 550 |
| BH | 10000 |
| BI | 6000 |

25

Test for ATLA specificity of antibody:

(1) Peptide A having various concentrations and ATLA samples described below were employed as samples.

ATLA positive sample

To $5 \times 10^9$ of cultured cells of ATLA positive cell line YAM [Science, 217, pp. 737—739 (1982)] 30 ml of a physiological saline solution was added followed by homogenization. Then the mixture was centrifuged ($105000 \times g$) for 1 hour to collect the supernatant. The amount of the proteins in the supernatant was adjusted with PBS to 10 mg/ml (the amount of the proteins was measured by a coloration method using a reagent for total protein assay made by the Otsuka Assay Research Laboratories, "Tonein-TP") (hereafter the supernatant is referred to as "YAM Supernatant").

ATLA Negative sample

Supernatants obtained by treating CCRF-CEM (Immunol. Commum., 9(8), pp. 731—734 (1980)) and BALL-1 (Nature, London, 267, pp. 843—844 (1977)) which were ATLA negative cell lines in a manner similar to above were employed·(these supernatants are hereafter referred to as "CEM Supernatant" and "BAL Supernatant", respectively).

Further, a 0.05M phosphate buffer (pH 7.4) containing 0.25% BSA, 5mM EDTA and 0.02% $NaN_3$ was employed as a standard diluting solution.

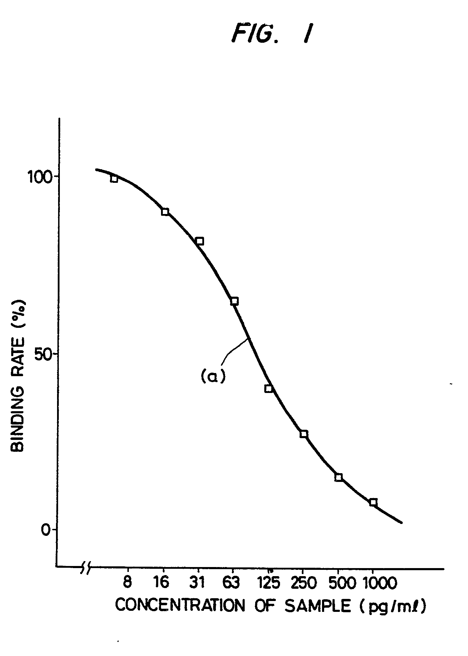

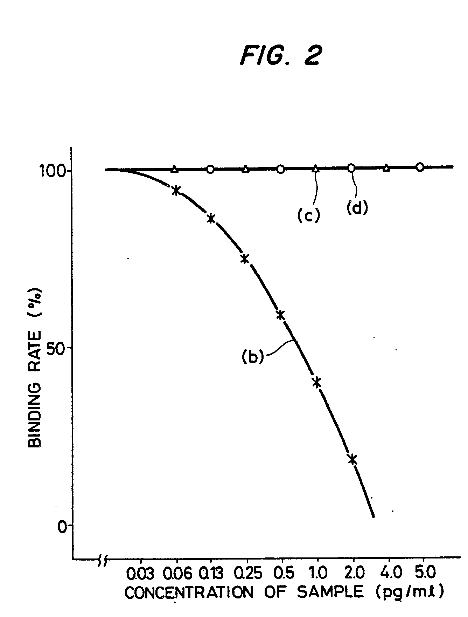

In each of test tubes, 0.2 ml of the standard diluting solution, 0.1 ml of a sample, 0.1 ml of Antibody A obtained in Preparation Example (of Antibody) 14 diluted so as to give a titer of 50,000 in finally obtained assay system and 0.1 ml of $^{125}$I-labelled peptide (a dilution obtained by diluting Labelled Peptide B obtained as described above to about 10000 cpm) were charged. After incubating the mixture at 4°C for 72 hours, 0.1 ml of normal porcine serum was added thereto. Then, 0.5 ml of a suspension of activated charcoal coated with dextran was added to the mixture. The mixture was allowed to stand for 30 minutes at 4°C. Thereafter, the mixture was centrifuged at 4°C for 30 minutes at 3000 rpm to separate the antibody-$^{125}$I-labelled peptide complex (B) from the unreacted (unbound) $^{125}$I-labelled peptide (F). The radioactive ray of the complex was counted to determine a percentage of (B) at each concentration and dilution of the respective samples. The results obtained are shown in Fig. 1 and Fig. 2.

In each of the figures, the vertical axis represents a binding % (B/Bo×100 wherein Bo is a percentage of (B) when the concentration of a sample is made 0) and the abscissa represents concentrations of samples (concentration of Peptide A and, protein contents of YAM Supernatant, CEM Supernatant and BALL Supernatant). In Fig. 1, Curve (a) represents Peptide A. In Fig. 2, Curves (b), (c) and (d) represent YAM Supernatant, CEM Supernatant and BALL Supernatant, respectively.

It is evident that Fig. 1 shows a high degree of the affinity of Antibody A to Peptide A and Fig. 2 shows a high selectivity of the antibody to ATLA. It is further evident from Fig. 2 that no cross to ATLA negative cell-induced proteins was observed with the antibodies of the present invention.

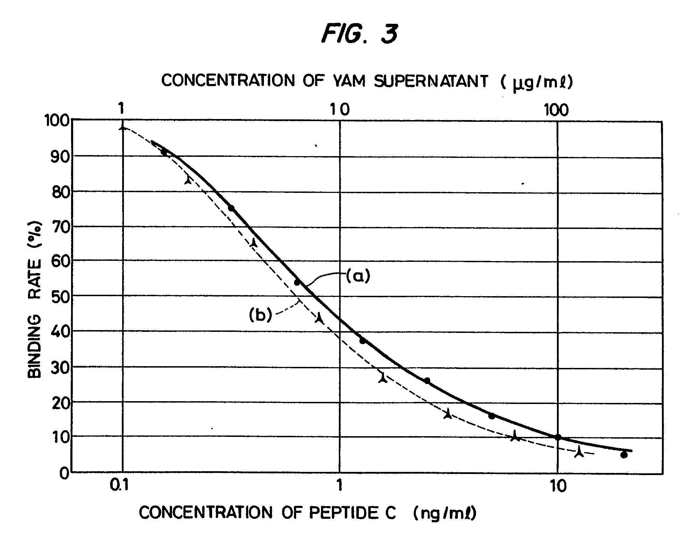

(2) In a manner similar to (1) above, ATLA specificity test was carried out. The results obtained are shown in Fig. 3 to 5.

In the figures, the vertical axis represents a binding % (B/Bo×100 wherein Bo is a percentage of (B) when the concentration of a sample is made 0) and the abscissa represents concentrations of the sample (concentration of Peptide C, F or H and, protein contents of YAM Supernatant).

Fig. 3 shows results of ATLA specificity test using Antibody X; curves (a) and (b) represent Peptide C and TAM Supernatant, respectively.

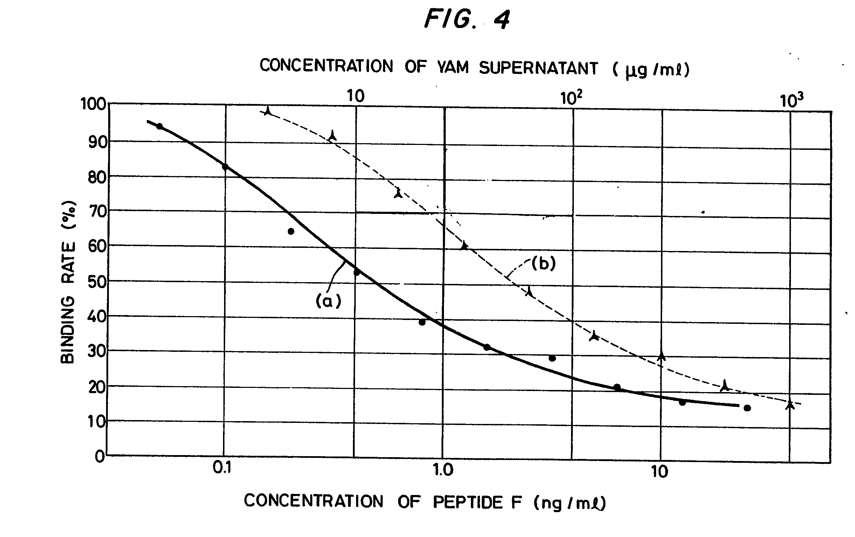

Fig. 4 shows results of ATLA specificity test using Antibody AA; curves (a) and (b) represent Peptide F and YAM Supernatant, respectively.

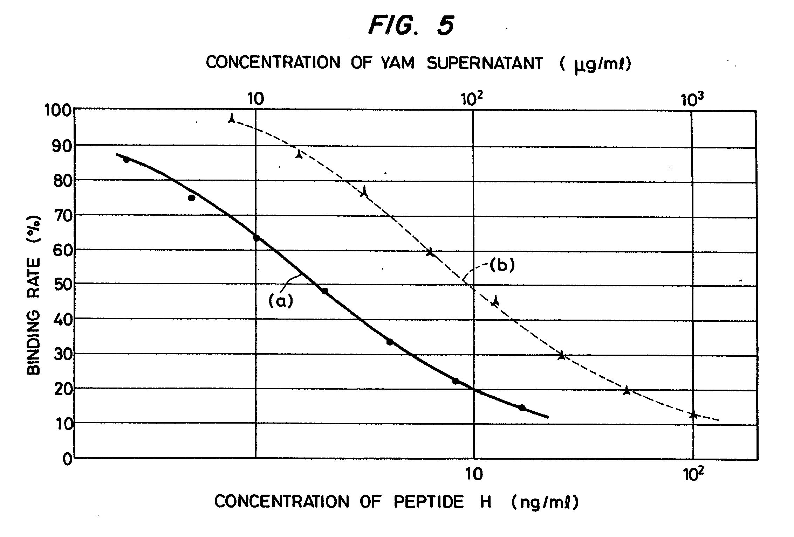

Fig. 5 shows results of ATLA specificity test using Antibody BF; curves (a) and (b) represent Peptide H and YAM Supernatant, respectively.

From these figures ATLA specificity of the antibody of the present invention can be seen.

## Claims

1. A human leukemia virus-related peptide selected from a peptide represented by the general formula (1):

$$R\text{-Pro-Val-Met-His-Pro-His-Gly-Ala-Pro-OH} \qquad (1)$$

wherein R is a hydrogen atom or a group of the formula H-Tyr-, in which the Tyr moiety may be labelled with radioactive iodine; a peptide represented by the formula (2):

$$H\text{-Tyr-Val-Glu-Pro-Thr-Ala-Pro-Gln-Val-Leu-OH} \qquad (2)$$

a peptide represented by the general formula (3):

26

EP 0 107 053 B1

R-Ile-Pro-His-Pro-Lys-Asn-Ser-Ile-Gly-Gly-Glu-Val-OH (3)

wherein R is the same as defined above;
a peptide represented by the general formula (4):

R-Thr-Trp-Thr-Pro-Lys-Asp-Lys-Thr-Lys-Val-Leu-OH (4)

wherein R is the same as defined above;
a peptide represented by the formula (5):

H-Val-Val-Gln-Pro-Lys-Lys-Pro-Pro-Pro-Tyr-OH (5)

in which the Tyr moiety may be labelled with radioactive iodine;
a peptide represented by the general formula (6):

R-Met-Gly-Gln-Ile-Phe-Ser-Arg-Ser-Ala-Ser-Pro-OH (6)

wherein R is the same as defined above; and
a peptide represented by the formula (7):

H-Tyr-Pro-Glu-Gly-Thr-Pro-Lys-Asp-Pro-Ile-Leu-Arg-Ser-Leu-Arg-Ser-Leu-OH (7)

in which the Tyr moiety may be labelled with radioactive iodine.
2. A peptide according to Claim 1, which is represented by the general formula (1):

R-Pro-Val-Met-His-Pro-His-Gly-Ala-Pro-OH (1)

wherein R is a hydrogen atom or a group of the formula, H-Tyr.
3. A peptide according to Claim 1, in which is represented by the formula (2):

H-Tyr-Val-Glu-Pro-Thr-Ala-Pro-Gln-Val-Leu-OH (2)

4. A peptide according to Claim 1, which is represented by the general formula (3):

R-Ile-Pro-His-Pro-Lys-Asn-Ser-Ile-Gly-Gly-Glu-Val-OH (3)

wherein R represents a hydrogen atom or a group of the formula H-Tyr.
5. A peptide according to Claim 1, which is represented by the general formula (4):

R-Thr-Trp-Thr-Pro-Lys-Asp-Lys-Thr-Lys-Val-Leu-OH (4)

wherein R represents a hydrogen atom or a group of the formula H-Tyr.
6. A peptide according to Claim 1, which is represented by the formula (5):

H-Val-Val-Gln-Pro-Lys-Lys-Pro-Pro-Pro-Tyr-OH (5)

7. A peptide according to Claim 1, which is represented by the general formula (6):

R-Met-Gly-Gln-Ile-Phe-Ser-Arg-Ser-Ala-Ser-Pro-OH (6)

wherein R represents a hydrogen atom or a group of the formula H-Tyr.
8. A peptide according to Claim 1, which is represented by the formula (7):

H-Tyr-Pro-Glu-Gly-Thr-Pro-Lys-Asp-Pro-Ile-Leu-Arg-Ser-Leu-OH (7)

9. A peptide according to Claim 1, which is represented by the formula

H-Tyr*-Pro-Val-Met-His-Pro-His-Gly-Ala-Pro-OH

wherein Tyr* represents Tyr labelled with radioactive iodine.
10. A peptide according to Claim 1, which is represented by the formula

H-Tyr*-Val-Glu-Pro-Thr-Ala-Pro-Gln-Val-Leu-OH

wherein Tyr* represents Tyr labelled with radioactive iodine

27

11. A peptide according to Claim 1, which is represented by the formula

H-Tyr*-Ile-Pro-His-Pro-Lys-Asn-Ser-Ile-Gly-Gly-Glu-Val-OH

wherein Tyr* represents Tyr labelled with radioactive iodine.

12. A peptide according to Claim 1, which is represented by the formula

H-Tyr*-Thr-Trp-Thr-Pro-Lys-Asp-Lys-Thr-Lys-Val-Leu-OH

wherein Tyr* represents Tyr labelled with radioactive iodine.

13. A peptide according to Claim 1, which is represented by the formula

H-Val-Val-Gln-Pro-Lys-Lys-Pro-Pro-Pro-Tyr*-OH

wherein Tyr* represents Tyr labelled with radioactive iodine.

14. A peptide according to Claim 1, which is represented by the formula

H-Tyr*-Met-Gly-Gln-Ile-Phe-Ser-Arg-Ser-Ala-Ser-Pro-OH

wherein Tyr* represents Tyr labelled with radioactive iodine.

15. A peptide according to Claim 1, which is represented by the formula

H-Tyr*-Pro-Glu-Gly-Thr-Pro-Lys-Asp-Pro-Ile-Leu-Arg-Ser-Leu-OH

wherein Tyr* represents Tyr labelled with radioactive iodine.

16. Antisera containing an antibody of a human leukemia virus-related peptide obtainable by centrifuging the blood collected from a mammal, to which an antigen prepared by reacting a human leukemia virus-related peptide selected from the peptides (1) to (7) as defined in claim 1 as a hapten, with a carrier in the presence of a hapten-carrier binding agent, has been administered.

17. The antibody-containing sera as claimed in Claim 16, wherein said carrier is selected from an animal serum albumin, an animal serum globulin, an animal thyroglobulin, an animal hemoglobulin, an animal hemocyanin, an ascaris extract, a polylysine, a polyglutamic acid, a lysine-glutamic acid copolymer, and a copolymer containing lysine or ornithine; and said hapten-carrier binding agent is selected from a diazonium compound, an aliphatic dialdehyde, a dimaleimide compound a maleimidocarboxyl - N - hydroxysuccinimide ester and a carbodiimide.

18. The antibody-containing sera as claimed in Claim 17, wherein said mammal is rabbit or guinea pig.

19. The antibody-containing sera as claimed in Claim 18, wherein said carrier is an ascaris extract, Keyhole limpet hemocyanin or bovine serum albumin and said hapten-carrier binding agent is glutaraldehyde N,N - dicyclohexylcarbodiimide or bisdiazotized benzidine.

20. A method for preparing an antibody of human leukemia virus-related peptide-containing sera comprising the following steps:

reacting a human leukemia virus-related peptide selected from the peptides (1) to (7) as defined in Claim 1 as a hapten, with a carrier in the presence of a hapten-carrier binding agent to form an antigen,

administering the resulting antigen to a mammal to form an antibody,

centrifuging the blood collected from the animals, and

isolating the antibody-containing serum from the blood.

**Patentansprüche**

1. Ein dem Humanleukämievirus verwandtes Peptid, ausgewählt aus einem Peptid der allgemeinen Formel (1)

R-Pro-Val-Met-His-Pro-His-Gly-Ala-Pro-OH          (1)

worin R ein Wasserstoffatom oder eine Gruppe der Formel H-Tyr- ist, in welcher die Tyr-Gruppe mit radioaktivem Jod markiert sein kann;

einem Peptid der Formel (2)

H-Tyr-Val-Glu-Pro-Thr-Ala-Pro-Gln-Val-Leu-OH          (2)

einem Peptid der allgemeinen Formel (3)

R-Ile-Pro-His-Pro-Lys-Asn-Ser-Ile-Gly-Gly-Glu-Val-OH          (3)

worin R die vorher angegegeben Bedeutung hat;

EP 0 107 053 B1

einem Peptid der allgemeinen Formel (4)

$$R\text{-Thr-Trp-Thr-Pro-Lys-Asp-Lys-Thr-Lys-Val-Leu-OH} \tag{4}$$

worin R die vorher angegebene Bedeutung hat;
einem Peptid der allgemeinen Formel (5)

$$H\text{-Val-Val-Gln-Pro-Lys-Lys-Pro-Pro-Pro-Tyr-OH} \tag{5}$$

in welcher der Tyr-Rest mit radioaktivem Jod markiert sein kann;
einem Peptid der allgemeinen Formel (6)

$$R\text{-Met-Gly-Gln-Ile-Phe-Ser-Arg-Ser-Ala-Ser-Pro-OH} \tag{6}$$

in welcher R die vorher angegebene Bedeutung hat; und
einem Peptid der allgemeinen Formel (7)

$$H\text{-Tyr-Pro-Glu-Gly-Thr-Pro-Lys-Asp-Pro-Ile-Leu-Arg-Ser-Leu-Arg-Ser-Leu-OH} \tag{7}$$

in welcher der Tyr-Rest mit radioaktivem Jod markiert sein kann.
2. Peptid gemäss Anspruch 1 der allgemeinen Formel (1)

$$R\text{-Pro-Val-Met-His-Pro-His-Gly-Ala-Pro-OH} \tag{1}$$

in welcher R ein Wasserstoffatom oder eine Gruppe der Formel H-Tyr ist.
3. Peptid gemäss Anspruch 1 der allgemeinen Formel (2)

$$H\text{-Tyr-Val-Glu-Pro-Thr-Ala-Pro-Gln-Val-Leu-OH} \tag{2}$$

4. Peptid gemäss Anspruch 1 der allgemeinen Formel (3)

$$R\text{-Ile-Pro-His-Pro-Lys-Asn-Ser-Ile-Gly-Gly-Glu-Val-OH} \tag{3}$$

in welcher R ein Wasserstoffatom oder eine Gruppe der Formel H-Tyr ist.
5. Peptid gemäss Anspruch 1 der allgemeinen Formel (4)

$$R\text{-Thr-Trp-Thr-Pro-Lys-Asp-Lys-Thr-Lys-Val-Leu-OH} \tag{4}$$

in welcher R ein Wasserstoffatom oder eine Gruppe der Formel H-Tyr ist.
6. Peptid gemäss Anspruch 1 der Formel (5)

$$H\text{-Val-Val-Gln-Pro-Lys-Lys-Pro-Pro-Pro-Tyr-OH} \tag{5}$$

7. Peptid gemäss Anspruch 1 der allgemeinen Formel (6)

$$R\text{-Met-Gly-Gln-Ile-Phe-Ser-Arg-Ser-Ala-Ser-Pro-OH} \tag{6}$$

in welcher R ein Wasserstoffatom oder eine Gruppe der Formel H-Tyr ist.
9. Peptid gemäss Anspruch 1 der Formel (7)

$$H\text{-Tyr-Pro-Glu-Gly-Thr-Pro-Lys-Asp-Pro-Ile-Leu-Arg-Ser-Leu-OH} \tag{7}$$

9. Peptid gemäss Anspruch 1 der Formel

$$H\text{-Tyr*-Pro-Val-Met-His-Pro-His-Gly-Ala-Pro-OH}$$

in welcher Tyr* ein mit radioaktivem Jod markiertes Tyr bedeutet.
10. Peptid gemäss Anspruch 1 der Formel

$$H\text{-Tyr*-Val-Glu-Pro-Thr-Ala-Pro-Gln-Val-Leu-OH}$$

in welcher Tyr* ein mit radioaktivem Jod markiertes Tyr bedeutet.
11. Peptid gemäss Anspruch 1 der Formel

29

H-Tyr*-Ile-Pro-His-Pro-Lys-Asn-Ser-Ile-Gly-Gly-Glu-Val-OH

in welcher Tyr* ein mit radioaktivem Jod markiertes Tyr bedeutet.

12. Peptid gemäss Anspruch 1 der Formel

H-Tyr*-Thr-Trp-Thr-Pro-Lys-Asp-Lys-Thr-Lys-Val-Leu-OH

in welcher Tyr* ein mit radioaktivem Jod markiertes Tyr bedeutet.

13. Peptid gemäss Anspruch 1 der Formel

H-Val-Val-Gln-Pro-Lys-Lys-Pro-Pro-Pro-Tyr*-OH

in welcher Tyr* ein mit radioaktivem Jod markiertes Tyr bedeutet.

14. Peptid gemäss Anspruch 1 der Formel

H-Tyr*-Met-Gly-Gln-Ile-Phe-Ser-Arg-Ser-Ala-Ser-Pro-OH

in welcher Tyr* ein mit radioaktivem Jod markiertes Tyr bedeutet.

15. Peptid gemäss Anspruch 1 der Formel

H-Tyr*-Pro-Glu-Gly-Thr-Pro-Lys-Asp-Pro-Ile-Leu-Arg-Ser-Leu-OH

in welcher Tyr* ein mit radioaktivem Jod markiertes Tyr bedeutet.

16. Antiserum, enthaltend eine Antikörper eines dem Humanleukämievirus verwandten Peptides, erhältlich durch Zentrifugieren des Blutes, das von einem Säugetier gesammelt wurde, dem ein Antigen, hergestellt durch Umsetzen eines Humanleukämievirus verwandten Peptides, ausgewählt aus Peptiden (1) bis (7) gemäss Anspruch 1, als ein Hapten mit einem Träger in Gegenwart eines Hapten - Trägerbindungsmittels, verabreicht worden ist.

17. Antikörper enthaltendes Serum gemäss Anspruch 16, bei dem der Träger ausgewählt ist aus einem Tierserumalbumin, einem Tierserumglobulin, einem Tierthyroglobulin, einem Tierhämoglobin, einem Tierhämocyanin, einem Ascarisextrakt, einem Polylysin, einer Polyglutaminsäure, einem Lysin - Glytaminsäure - Copolymer und einem Copolymer, enthaltend Lysin oder Ornithin; und das Hapten - Trägerbindungsmittel ausgewählt ist aus einer Diazoniumverbindung, einem aliphatischen Dialdehyd, einer Dimaleinsäureimid - Verbindung, einem Maleimidocarboxyl - N - hydroxysuccinimidester und einem Carbodiimid.

18. Antikörper enthaltende Sera gemäss Anspruch 17, bei denen das Säugetier ein Kaninchen oder ein Meerschweinchen ist.

19. Antikörper enthaltende Sera gemäss Anspruch 18, bei denen der Träger ein Ascarisextrakt, ein Keyhole limpet-Hämocyanin oder Rinderserumalbumin ist und das Hapten - Trägerbindungsmittel Glutaraldehyd, N,N - Dicyclohexylcarbodiimid oder bisdiazotiertes Benzidin ist.

20. Verfahren zur Herstellung eines Antikörpers von Humanleukämievirus verwandten Peptid enthaltenden Sera, umfassend folgende Stufen:

Umsetzen eines Humanleukämievirus verwandten Peptides, ausgewählt aus Peptiden (1) bis (7) gemäss Anspruch 1 als ein Hapten mit einem Träger in Gegenwart eines Hapten - Trägerbindungsmittels unter Ausbildung eines Antigens;

Verabreichen des gebildeten Antigens an ein Säugetier unter Ausbildung eines Antikörpers;

Zentrifugieren des von dem Tier gesammelten Blutes; und

Isolieren des Antikörper enthaltenden Serums aus dem Blut.

**Revendications**

1. Peptide apparenté au virus de la leucémie humaine choisi parmi un peptide représenté par la formule générale (1):

R-Pro-Val-Met-His-Pro-His-Gly-Ala-Pro-OH          (1)

dans laquelle R est un atome d'hydrogène ou un groupe de formule H-Tyr-, dans lequel le fragment Tyr peut être marqué avec de l'iode radioactif;

un peptide représenté par la formule (2):

H-Tyr-Val-Glu-Pro-Thr-Ala-Pro-Gln-Val-Leu-OH          (2)

un peptide représenté par la formule générale (3):

R-Ile-Pro-His-Pro-Lys-Asn-Ser-Ile-Gly-Gly-Glu-Val-OH (3)

dans laquelle R est comme défini ci-dessus;
un peptide représenté par la formule générale (4):

R-Thr-Trp-Thr-Pro-Lys-Asp-Lys-Thr-Lys-Val-Leu-OH (4)

dans laquelle R est comme défini ci-dessus;
un peptide représenté par la formule (5):

H-Val-Val-Gln-Pro-Lys-Lys-Pro-Pro-Pro-Tyr-OH (5)

dans laquelle le fragment Tyr peut être marqué avec de l'iode radioactif;
un peptide représenté par la formule générale (6):

R-Met-Gly-Gln-Ile-Phe-Ser-Arg-Ser-Ala-Ser-Pro-OH (6)

dans laquelle R est comme défini ci-dessus; et
un peptide représenté par la formule (7):

H-Tyr-Pro-Glu-Gly-Thr-Pro-Lys-Asp-Pro-Ile-Leu-Arg-Ser-Leu-Arg-Ser-Leu-OH (7)

dans laquelle le fragment Tyr peut être marqué avec de l'iode radioactif.
2. Peptide selon la revendication 1, qui est représenté par la formule générale (1):

R-Pro-Val-Met-His-Pro-His-Gly-Ala-Pro-OH (1)

dans laquelle R est un atome d'hydrogène ou un groupe de formule H-Tyr.
3. Peptide selon la revendication 1, qui est représenté par la formule (2):

H-Tyr-Val-Glu-Pro-Thr-Ala-Pro-Gln-Val-Leu-OH (2)

4. Peptide selon la revendication 1, qui est représenté par la formule générale (3):

R-Ile-Pro-His-Pro-Lys-Asn-Ser-Ile-Gly-Gly-Glu-Val-OH (3)

dans laquelle R représente un atome d'hydrogène ou un groupe de formule H-Tyr.
5. Peptide selon la revendication 1, qui est représenté par la formule générale (4):

R-Thr-Trp-Thr-Pro-Lys-Asp-Lys-Thr-Lys-Val-Leu-OH (4)

dans laquelle R représente un atome d'hydrogène ou un groupe de formule H-Tyr.
6. Peptide selon la revendication 1, qui est représenté par la formule (5):

H-Val-Val-Gln-Pro-Lys-Lys-Pro-Pro-Pro-Tyr-OH (5)

7. Peptide selon la revendication 1, qui est représenté par la formule générale (6):

R-Met-Gly-Gln-Ile-Phe-Ser-Arg-Ser-Ala-Ser-Pro-OH (6)

dans laquelle R représente un atome d'hydrogène ou un groupe de formule H-Tyr.
8. Peptide selon la revendication 1, qui est représenté par la formule (7):

H-Tyr-Pro-Glu-Gly-Thr-Pro-Lys-Asp-Pro-Ile-Leu-Arg-Ser-Leu-OH (7)

9. Peptide selon la revendication 1, qui est représenté par la formule

H-Tyr*-Pro-Val-Met-His-Pro-His-Gly-Ala-Pro-OH

dans laquelle Tyr* représente Tyr marqué avec de l'iode radioactif.
10. Peptide selon la revendication 1, qui est représenté par la formule

H-Tyr*-Val-Glu-Pro-Thr-Ala-Pro-Gln-Val-Leu-OH

dans laquelle Tyr* représente Tyr marqué avec de l'iode radioactif.

11. Peptide selon la revendication 1, qui est représenté par la formule

H-Tyr*-Ile-Pro-His-Pro-Lys-Asn-Ser-Ile-Gly-Gly-Glu-Val-OH

dans laquelle Tyr* représente Tyr marqué avec de l'iode radioactif.

12. Peptide selon la revendication 1, qui est représenté par la formule

H-Tyr*-Thr-Trp-Thr-Pro-Lys-Asp-Lys-Thr-Lys-Val-Leu-OH

dans laquelle Tyr* représente Tyr marqué avec de l'iode radioactif.

13. Peptide selon la revendication 1, qui est représenté par la formule

H-Val-Val-Gln-Pro-Lys-Lys-Pro-Pro-Pro-Tyr*-OH

dans laquelle Tyr* représente Tyr marqué avec de l'iode radioactif.

14. Peptide selon la revendication 1, qui est représenté par la formule

H-Tyr*-Met-Gly-Gln-Ile-Phe-Ser-Arg-Ser-Ala-Ser-Pro-OH

dans laquelle Tyr* représente Tyr marqué avec de l'iode radioactif.

15. Peptide selon la revendication 1, qui est représenté par la formule

H-Tyr*-Pro-Glu-Gly-Thr-Pro-Lys-Asp-Pro-Ile-Leu-Arg-Ser-Leu-OH

dans laquelle Tyr* représente Tyr marqué avec de l'iode radioactif.

16. Antisérums contenant un anticorps contre un peptide apparenté au virus de la leucémie humaine, pouvant être obtenus par centrifugation du sang recueilli à un mammifère auquel un antigène, préparé par réaction d'un peptide apparenté au virus de la leucémie humaine, choisi parmi les peptides (1) à (7) comme défini dans la revendication 1 comme haptène, avec un support en présence d'un agent de liaison haptène-support, a été administré.

17. Sérums contenant un anticorps selon la revendication 16, où ledit support est choisi parmi une sérum-albumine d'animal, une sérum-globuline d'animal, une thyroglobuline d'animal, une hémoglobuline d'animal, une hémocyanine d'animal, un extrait d'ascaris, une polylysine, un acide polyglutamique, un copolymère de lysine-acide glutamique et unc opolymère contenant la lysine ou l'ornithine; et ledit agent de liaison haptène-support est choisi parmi un composé de diazonium, un dialdéhyde aliphatique, un composé de dimaléimide, un ester de type maléimidocarboxyl - N - hydroxysuccinimide et un carbodiimide.

18. Sérums contenant un anticorps selon la revendication 17, où ledit mammifère est un lapin ou un cobaye.

19. Sérums contenant un anticorps selon la revendication 18, où ledit support est un extrait d'ascaris, l'hémocyanine de patelle ou la sérum-albumine bovine et ledit agent de liaison haptène-support est le glutaraldéhyde, le N,N - dicyclohexylcarbodiimide ou la benzidine bisdiazotée.

20. Procédé pour préparer des sérums contenant un anticorps contre un peptide apparenté au virus de la leucémie humaine, comprenant les stades suivants:

réaction d'un peptide apparenté au virus de la leucémie humaine, choisi parmi les peptides (1) à (7) comme définie dans la revendication 1, comme haptène, avec un support en présence d'un agent de liaison haptène-support pour former un antigène,

administration de l'antigène obtenu à un mammifère pour former un anticorps,

centrifugation du sang recueilli aux animaux et

isolement à partir du sang du sérum contenant un anticorps.

# FIG. 1

## FIG. 2

## FIG. 3

CONCENTRATION OF YAM SUPERNATANT ( μg/mℓ)

CONCENTRATION OF PEPTIDE C (ng/mℓ)

BINDING RATE (%)

(a)

(b)

EP 0 107 053 B1

## FIG. 4

CONCENTRATION OF YAM SUPERNATANT ( μg /mℓ)

EP 0 107 053 B1

## FIG. 5

CONCENTRATION OF YAM SUPERNATANT ( μg/ml)

(a)

(b)

BINDING RATE (%)

CONCENTRATION OF PEPTIDE H (ng/ml)

EP 0 107 053 B1